# EUROPEAN PATENT SPECIFICATION

(11) **EP 0 305 399 B1**
(45) Date of publication and mention of the grant of the patent: **23.11.1994**
(21) Application number: 87903558.2
(22) Date of filing: 04.05.1987
(51) Int. Cl.: C12Q 1/68, C12N 15/00

(54) **CHAOTROPIC METHOD FOR EVALUATING NUCLEIC ACIDS IN A BIOLOGICAL SAMPLE**
CHAOTROPISCHES VERFAHREN ZUR SCHÄTZUNG VON NUKLEINEN SÄUREN IN EINER BIOLOGISCHEN PROBE
PROCEDE CHAOTROPIQUE D'EVALUATION D'ACIDES NUCLEIQUES DANS UN SPECIMEN BIOLOGIQUE

(30) Priority: 02.05.1986 US 859003
(43) Date of publication of application: 08.03.1989
(73) Proprietor: HAHNEMANN UNIVERSITY, Philadelphia, PA 19102-1192 (US)
(72) Inventor: GILLESPIE, David, Glenmore, PA 19343 (US)
(74) Representative: W.P. THOMPSON & CO.
(86) International application number: US8701023
(87) International publication number: WO8706621

(56) References cited:
- US-A- 4 427 580
- US-A- 4 483 920
- FEBS LETTERS, vol. 155, no. 1, May 1983, Elsevier/North-Holland Biomedical Press, Amsterdam (NL); COX et al., pp. 73-78#
- BIOESSAYS, vol. 1, no. 6, 1984, Cambridge University Press, GB; GILLESPIE et al., pp. 272-276#
- CELLS, DNA, vol. 2, no. 3, 1983, Mary Ann Liebert Inc., New York, NY (US); BRESSER et al., pp. 243-253#
- ANALYTICAL BIOCHEMISTRY, vol. 129, 1983, Academic Press Inc., New York, NY (US); BRESSER et al., pp. 357-364#
- JOURNAL OF BIOLOGICAL CHEMISTRY, vol. 257, 1972, American Society of Biological Chemists Inc., Baltimore, MD (US); WHITE et al., pp. 8659-8672#
- PROCEEDINGS OF THE NATL. ACADEMY OF SCIENCES USA, vol. 77, no. 9, September 1980, National Academy of Sciences, Washington, DC (US); P.S. THOMAS, pp. 5201-5205#
- JOURNAL OF MOLECULAR BIOLOGY, vol. 12, 1965, Academic Press, New York, NY (US); GILLESPIE et al., pp. 829-842#

## Description

This invention relates to the fields of analytical chemistry and medicine, and in particular relates to a novel method for evaluating nucleic acids in a biological sample wherein said nucleic acids are made available for evaluation by chaotropic solubilization. The nucleic acids are evaluated by means of molecular hybridization with a complementary nucleic acid probe, in the chaotropic solution.

US-A-4483920 relates to the immobilisation of message RNA onto filter material by solubilising cellular components with a chaotropic salt and passing the resultant components through a filter.

D. Gillespie *et al, BioEssays* **1(6):** 272-275 refers to the role of chaotropic salts in two-phase gene diagnosis.

R A Cox and N J Smulian *FEBS Letters* **155(1):** 73-79 (May 1983) refers to a single step procedure for the isolation of individual mRNA species from crude lysates of *Physarum polycephalum.*

J Bresser *et al, DNA* **2(3):** 243-253 (1983) refers to quick=blot methods of immobilising mRNA or DNA onto nitrocellulose.

J Bresser and D Gillespie *Analytical Biochemistry* **129:** 357-364 (1983) refers to a procedure for quantitatively binding covalently closed circular DNA to nitrocellulose.

### 1. General Considerations

Tissues of patients are customarily examined for "markers" which may indicate a disease state. Marker evaluation can be an important part of a patient's initial diagnosis as well as providing a continued measurement of a patient's response to treatment and future prognosis. Conventional tissue markers include cell morphology and metabolism, the presence of certain enzymic activities or proteins or other molecules of biological import, the accumulation or disappearance of such molecules, etc. Recently, gene structure has provided a marker for certain diseases (Geever et al., PNAS 78:5081-5085, 1981; Orkin et al., N. Eng. J. Med. 299:166-172; 1981, Chang and Kan, PNAS 76:2886-2889, 1979; Philips et al., PNAS 77:2853-2856, 1980).

Until this invention, however, it has been relatively difficult to easily and economically measure primary gene activation, i.e., the accumulation of specific message RNA molecules (RNA transcription). Existing procedures generally employ an appropriate "probe," usually a radiolabelled DNA molecule of known nucleotide sequence, to measure a quantity of specific (complimentary) message RNA sequence by a process called "molecular hybridization" (Gillespie, D., Methods Enzymology 12 B:641-668, 1968). Such procedures usually require first purifying message RNA from cells or tissues, a costly and laborious process. Then, molecular hybridization of the purified RNA to the probe is effected, and can be aided by immobilizing the RNA on a solid surface (Gilham, P.T., Biochemistry 7:2809-2813, 1968; Ponian, M.S., et al., Biochemistry 10:424-427, 1971; Wagner, A.F. et al., BBRC 45:184-189, 1971; Sheldon, R. et al., PNAS 69:417-421, 1972; Saxinger, W.C. et al., PNAS 69:2975-2978, 1972; Noyes, B.E. and Stark, G.R., Cell 5:301-310, 1975; Alwine, J.C. et al., PNAS 74:5350-5354, 1977; Thomas P.S., PNAS 77:5201-5205). In any case, RNA purification apart from immobilization is required by these procedures.

Alternatively, cells can be deposited on microscope slides or a like surface and molecular hybridization can be performed upon the message RNA in the cells, a technique called "in situ molecular hybridization" (Pardue, M.L. and Gall, J.G., PNAS 64:600-604, 1969; Brahic, M. and Haase, A.T., PNAS 75:6125-6129, 1978; Angerer, L.M. and Angerer, R.C., Nuc. Ac. Res. 9:2819-2840, 1981). However, this process can be time-consuming and unreliable and is only useful when the assay can be performed upon single cells.

### 2. Description of the Background Materials

### A. DNA Immobilization in NaCl.

Following is a brief overview of the history of attempts to immobilize DNA on solid supports.

Since 1977 our ability to manipulate DNA and our knowledge of its structure has advanced in enormous leaps. This has been made possible primarily because of the technology of DNA immobilization and, more recently, because of the ability to sequence DNA. Since the original work on the binding of purified, single-stranded DNA to nitrocellulose (NC) membrane by Nygaard and Hall (Biophys. Biochem. Res. Comm. 12:98-104, 1963) and by Gillespie and Spiegelman (J. Mol. Biol. 12:829-842, 1965), DNA-NC has been exploited in an array of imaginative and elegant ways.

One of the most imaginative extensions was devised by Grunstein and Hogness (Proc. Nat. Acad. Sci. USA 72:3961-3965, 1975) who discovered how to blot colonies of whole cells onto NC in such a way that only their DNA stuck to the membrane, providing a DNA image of the original colony of the membrane. This "colony lift" technique permitted the rapid screening of recombinant organism and advanced the present state of gene cloning by some 5-10 years. However, this method and others based on the same principle (e.g. Scotto et al., Hepatology 3:279-284, 1983) are not quantitative because the conditions used to cause DNA denaturation (NaOH) interfere with DNA immobilization.

One of the most elegant extensions of DNA immobilization was invented by Southern (J. Mol. Biol. 98:503-517, 1975). He deduced a means to transfer to NC DNA which had been purified and then fractionated according to size in agarose gels. He was able to produce a DNA image of the gel-fractionated DNA on NC, which could then be probed for selected sequences. The "Southern transfer" or "Southern blot" is probably the most important technique we have available today for comparative gene structure analysis, short of DNA sequencing.

### B. RNA Immobilization in NaCl.

Following is a brief overview of the history of attempts to immobilize RNA on solid supports.

In early DNA immobilization work, Gillespie and Spiegelman (J. Mol. Biol. 12:829-842, 1965) categorically reported that RNA would not bind to NC. Some years later, DeLarco and Guroff (Biochem. Biophys. Res. Comm. 50:486-492, 1973) corrected this erroneous conclusion by immobilizing RNA to NC, using very concentrated NaCl solutions to do this. They thought that only certain RNA molecules stuck to NC, specifically RNA molecules bearing a terminal tract of adenosine residues, the so-called "poly(A) tail." All RNA molecules with sufficiently long poly(A) (herein also referred to as "poly(A)-plus RNA") tails could bind to NC in concentrated NaCl. As far as we know, all of these polyadenylated RNA molecules were message RNA, capable of coding for proteins. RNA molecules lacking poly(A) (herein also referred to as "poly(A)-minus RNA") did not appear to interact with NC in concentrated NaCl. Most of this poly(A)-minus RNA is ribosomal RNA and transfer RNA, which are noncodogenic RNA species, although a few percent of the poly(A)-minus RNA is "tailless" message RNA. Therefore, the concentrated-NaCl method for binding purified RNA to NC was selective for message RNA but it did not include all message RNA species.

In the mid and late 1970s several supports for immobilizing mRNA were described (see Seed B. Genetic Engineering 4:91-102, 1982 for review). These were papers of various kinds which displayed reactive groups to which pure RNA could be covalently attached. These papers were not selective for mRNA, or even for RNA, since DNA and proteins could also be attached to the reactive groups. However, these did have the advantage that all RNA molecules, not a select population, could be immobilized on them.

In 1979, Pat Thomas reported an important observation. She noted that denatured RNA of all kinds could be immobilized onto NC in concentrated NaCl solutions (Proc. Nat. Acad. Sci. USA 77:5201-5205, 1980). She exploited this observation by developing the RNA-analogue of the Southern transfer, a method quite naturally dubbed the "Northern Transfer." Pat Thomas' work introduced a new explanation for the DeLarco/Guroff results; namely, that the RNA which bound to NC in concentrated NaCl did not do so through its long poly(A) tail, but because it was naturally more denatured than RNA with short or not terminal poly (A).

Unfortunately, the Northern transfer methodology has not lent itself to the immobilization of mRNA from whole cells or other natural produce biological sources. White and Bancroft (J. Biol. Chem. 257:8659-8572, 1982) were able to develop a method for immobilizing total RNA from cytoplasmic extracts based on Pat Thomas' denaturation procedure but it has not become popular, probably because of the enormous problem presented by protein coimmobilization and the nagging question of how much denatured DNA would co-immobilize. A minor advance was made when it was learned that in NaCl, sodium dodecyl sulfate suppresses DNA-NC and protein-NC interactions but does not interfere with mRNA-NC interactions (Bresser et al., DNA 2:243-254, 1983), but the techniques based on selectively-through-detergents have also fallen short of reliable quantitation and all are restricted to the use of small amounts of cellular material.

All of the procedures described above depended on NaCl-promoted interactions between NC and DNA or RNA. In all these cases the nucleic acid had to be "fixed" to the NC by baking at high temperature. Consequently, RNA adsorbed to NC was biologically inactive--it could not be reverse transcribed or translated.

Gillespie et al., U.S. Patent No. 4,483,920, issued on November 20, 1984, describe a method for directly immobilizing an mRNA from cells onto filter paper wherein cellular mRNA is solubilized with a chaotropic salt, immobilized on a filter which selectively binds mRNA, with an optional baking step.

The thus immobilized target mRNA is then hybridized with a labeled DNA probe.

Cox et al., FEBS LETTERS, Vol. 155, No. 1, 73-80 (May, 1983) describe a single-step procedure for the isolation of a particular mRNA from crude lysates of Physarum polycephalum, exposed by solubilization with, among others, guanidinium isothiocyanate by hybridization to complementary DNA which has been immobilized by binding to aminotriophenol paper. Thus not just the poly(A)-tails, but the coding region of the mRNA was isolated by molecular hybridization in the crude guanidinium isothiocyanate lysate.

Strayer et al., PNAS, Vol. 80, pp. 4770-4774 (August, 1983) disclose liquid-liquid molecular hybridization of chaotropically solubilized DNA where the hybridization is effected in the chaotropic solution. The duplex regions lowered the buoyancy of the DNA complex in direct proportion to the length ratio of the double-stranded to single-stranded elements, thus permitting analysis of the DNA by means of a NaI gradient.

Kohne, International Application No. WO 84/02721, published July 19, 1984, disclosed a method for quantitating and detecting RNA-containing organisms comprising solubilizing the nucleic acid of said organism and probing the solubilized nucleic acid with a labeled probe complementary to the nucleic acid, said hybridization effected in solution, without purifying the sample RNA. At page 34, Kohne suggests using a chaotropic agent as the solubilizing agent. Kohne utilizes an "in solution" hybridization, i.e., both the probe and the sample RNA were in solution.

Cox et al., European Patent Application Publication No. 0 127 327 A1, published on December 5, 1984, disclose an assay for nucleic acids comprising chaotropically solubilizing cellular nucleic acid and performing a molecular hybridization in the chaotropic solution utilizing labeled probe complementary to the target nucleic acid. While all the examples disclosed by Cox et al. are directed to heterogeneous hybridization utilizing immobilized, labeled probe, at page 7, line 18, Cox et al. do suggest that the hybridization might be "homogenous." No homogeneous hybridization parameters are disclosed.

However, prior to the effective date of this invention, a need had continued to exist for a means for evaluating the nucleic acid of a biological sample that permitted molecular hybridization between a labeled nucleic acid probe complementary to the sample nucleic acid and the sample nucleic acid which did not require a prior purification and/or immobilization of the solubilized sample nucleic acid.

More importantly, a need had continued to exist for a method for evaluating cellular nucleic acids which is adapted for clinical use. Such a method must retain the accuracy and sensitivity of the technical procedure cited above when used in the research laboratory. However, clinical adaptation additionally demands speed, simplicity/reliability, economy, versatility and automatability.

Speed - An ideal clinical test should be carried out in 30 min., i.e., during an outpatient's visit to the doctor's office. Minimally, a gene diagnosis test should be more rapid than competing culture, immunological or biochemical tests. Practically, a 2-3 hour goal for completion of a gene diagnosis test has been set by most commercial houses.

Simplicity/Reliability - A clinical test should be able to be performed by a laboratory technician with a high school degree. The method should not be perturbed by ordinary fluctuations in temperature, time, volumes, sample composition, etc., expected in a clinical laboratory. The method should be simple enough to automate.

Economy - A gene diagnosis test adapted to the clinic must be as economical as the test it replaces in the clinical market, typically under ten dollars per test.

Versatility - A gene diagnosis method should be applicable to the wide range of "dirty" clinical samples including blood, urine, solid tissues, stools, swabs, etc., containing viruses, cells, microorganisms, etc.

Automatability - All or portions of gene diagnosis procedures will be done by machine in clinical laboratories. Such a procedure will have a minimum of steps and will be performed at near ambient temperatures without corrosive or toxic solvents or solutes. Such a procedure will avoid cumbersome methods such as centrifugation and gel electrophoresis.

Clinical adaptability demands that all of the above qualities be added to laboratory-grade accuracy and sensitivity. To our knowledge, the prior art does not anticipate how this is to be done.

### C. Measurement of HIV Load in Patients

AIDS patients were found to have antibodies which cross-react with HTLV-I (Essex et al., Science 220:859 (1983)). Later, a new virus, HIV or HTLV III/LAV (AIDS virus) was discovered (Barre-Sinoussi et al., Science 220:868 (1983); Povovic et al., ibid 224:497 (1984)), and it was shown that AIDS patients have antibodies against various proteins of AIDS virus (Sarngadharan et al., Science 224:506 (1984); Kalyanaraman et al., Science:225:321 (1984)). AIDS patients carry AIDS virus DNA sequences in cells (Hahn et al., Nature 312:166 (1984); Shaw et al., Science 227:177 (1985)) and in brain cells (Shaw et al., Science 227:177 (1985)). Very few lymphocytes may contain AIDS virus (Hahn et al., Nature 312:166 (1984); Shaw et al., Science 226:1165 (1984); Shaw et al., Science 227:177 (1985)). The virus is cytopathic to T helper cells but may be carried by other blood cells without cell lysis (D. Morgan and J. Levy, personal communication).

Recent estimates suggest that 1 lymphocyte in 10⁴ or fewer may be infected. AIDS virus isolates from different individuals can exhibit genomic diversity (Wong-Staal et al., Science 229:759 (1985)). The AIDS virus is genetically related to lentivirus (Chiu et al., Nature 317:366 (1985)) and, like lentiviruses, is cytopathic, at least in T4 cells. Since lentiviruses can escape immune surveillance, Chill et al. "emphasize the challenge that these rapidly evolving retroviruses present in prevention and control of their associated disease."

Many laboratories and commercial firms are attempting to measure HIV nucleic acids in AIDS patients. Cherman, Gallo, Volsky and others have prepared clones of HIV genes for use as probes. Measurements of HIV nucleic acids have been made following purification of RNA or DNA from cells infected with virus in vitro and from blood cells, but the techniques are neither rapid nor simple enough to use routinely on clinical samples. Detection of HIV RNA has been done by in situ hybridization following deposition of blood cells on microscope slides (Shaw et al., Science 227:177 (1985)). This approach is being exhibited by ENZO Biochemicals, ONCOR and DuPont in the form of commercially available kits or probes; however, it is clear that this approach will not offer the simplicity or reliability required of a clinically valuable HIV test. There is no convincing evidence that in situ hybridization can measure HIV quantitatively nor that a confirmatory test exists for cases of apparently positive detection. "Dot blots" have been used to measure virus RNA or DNA in infected cells, without nucleic acid purification. RNA can be selectively immobilized from lysed cells in NaI, GuSCN, formaldehyde/SDS or SDS; however, the efficiency of RNA immobilization is variable. Hybridization to immobilized nucleic acids RNA is relatively slow. A further defect of immobilization of nucleic acids from crude lysates is that the coimmobilization of proteins interferes with hybridization. All in all, these immobilization approaches, whether using lysates or purified nucleic acids, have lacked the sensitivity required of a clinically valuable HIV assay. Cetus has employed the strategy of target amplification, but presently this can only be performed with purified nucleic acids and presently requires a DNA target.

The current tests for detection of HIV virus is a coculture procedure. Mononuclear blood cells from AIDS patients are cocultured with sensitive indicator cells, usually PHA stimulated lymphocytes from normal volunteer. After 1-3 weeks of coculture, virus released into the medium is assayed by measuring reverse transcriptase or viral antigen. The sensitivity of the present coculture tests is not known. Variation in the isolation frequency of successive cultures or certain patients has been observed. This variation might be related to the susceptibility of different donor preparations of human lymphocytes to infection with HIV (T. Folks et al., J. Immunol. 136:4049 (1985)). It is not yet possible to quantitate virus load with the coculture assay.

Work is in progress at Abbot and DuPont and elsewhere to develop a direct test for HIV antigens in serum of ARC/AIDS patients. Assays performed at Abbott on ARC/AIDS samples showed only 50% positivity by the direct assay. It can be anticipated, additionally, that direct antigen tests will be complicated by the simultaneous presence of small or amounts of antibodies in ARC/AIDS patients which form complexes with the antigen and are cleaved from the blood (J. Goudsmit et al., The Lancet, Sat 26 July, p. 177-180 (1986)).

### SUMMARY OF THE INVENTION

The invention relates to a method of detecting and/or quantifying target nucleic acid in a biological sample, the method comprising:
A. solubilizing the nucleic acid of the biological sample by contacting the biological sample with a chaotropic salt, whereby a solution of solubilized nucleic acid is produced;
B. incubating this solution of solubilized nucleic acid with at least one nucleic acid probe complementary to at least a portion of the solubilized nucleic acid, under conditions which promote molecular hybridization between the probe and the solubilized nucleic acid; and
C. detecting the molecular hybridization.

The present invention thus provides a significant improvement in disease diagnosis. The invention is based on a method for immobilizing mRNA from a biological source which is simple, fast, and accurate. Broadly, this method relates to a process for immobilizing RNA, the process comprising the steps of:
A. dissolving a biological source containing RNA by contacting said biological source with a chaotropic salt solution; and
B. filtering the thus dissolved biological source through a filter material which selectively immobilizes said mRNA.

The invention is quite advantageous relative to existing methodology which, as previously mentioned, requires a purification step and, separately, then requires a immobilization step. By the present invention a biological source containing RNA, such as cells, is first dissolved in a chaotropic salt solution which forms a solution of the mRNA and the rest of the cellular matter. By then filtering the solution of cellular material through a filter selectively immobilizes the mRNA, the rest of the cellular material is accordingly dispensed with by passing through the filter. Thus a great advantage of the invention is that the immobilization step is also inherently the purification step. No separation of the purification step from the immobilization step is required.

The fact that the immobilization step is also the purification step represents an important advance so far as simplifying and improving existing methods is concerned. Ordinarily biological samples must be purified extensively in order to remove co-immobilizing contaminants so that immobilization of a desired component can take place at all. In the present invention, however, the use of a chaotropic salt solution to dissolve biological samples results in the component of interest, RNA (or DNA as hereinafter explained) being selectively immobilized on an immobilizing filter material.

By "selectively immobilizing" RNA from a dissolved source of RNA such as cells or bacteria, it is meant that the majority of extraneous (i.e. non-RNA) material passes through the immobilization filter, although co-immobilization of some small fraction of non-RNA material is probably unavoidable. Simply passing the dissolved (i.e., in a chaotropic solution) source of RNA provides as much, if not more, purification than existing procedures employing separate purification steps. The preferred steps, in the present invention are (A) and (B) above, although the invention can advantageously be rendered even more effective by the inclusion of auxiliary (i.e., non-essential) process steps, as hereinafter described.

A "chaotropic" salt is one which dissolves a biological source of RNA (such as cells and bacteria) and renders the RNA therein in a form suitable for selective immobilization, primarily by interrupting weak intramolecular forces generally known as van der Waal's attractions. Van der Waal's attractions, for example, are primarily responsible for holding fatty (e.g., lipid bilayer type) cellular membranes together. Chaotropic salts also solvate other types of biological molecules such as proteins. Chaotropic salts perform several functions simultaneously which make them particularly suitable for use in the present invention:
1. they dissolve cells and cellular components or other mRNA sources;
2. they tend to denature nucleic acids;
3. they allow selective nucleic acid binding to a suitable immobilizing surface.

Chaotropic salts form a reasonably select subset within all known salts. Certainly, not all salts are chaotropic and, therefore, are not suitable for use in the present invention. Chaotropic efficacy is characterized physically by a biological source substantially completely dissolving in chaotropic salt solution. Dissolution, for purposes of the invention, can be measured in terms of the amount of visible light scattered by particulate (i.e., undissolved) material). A chaotropic salt generally dissolves a biological sample to produce a substantially clear solution (although the solution may be colored). "Dissolution" may be defined as a reduction by a factor of at least about 2 in the optical density of visible light (e.g., measured at 600 millimicrons) measured for a chaotropically dissolved biological sample, relative to the optical density measured for an identical sample suspended in 5% glycerol in water. "Dissolution", for any specific salt may be operationally determined by mixing (mechanically as known in the art) one milligram of cells for each millilitre of solution at a predetermined salt concentration. For operational purposes of determining whether a salt is chaotropic, the predetermined concentration is 5 molar, although for use with actual samples the concentration can be varied to suit the nature of the biological source from which the sample is derived. If the salt is chaotropic, mixing results in "dissolution" as indicated by at least about a 2-fold reduction, relative to a suspension in 5% glycerol in water, of scattered light measured in a standard turbidimeter. A numerical illustration of this may be found in Example 7.

Suitable chaotropic salts include alkali metal perchlorates, iodides, trifluoroacetates, trichloroacetates and thiocyanates. Specific chaotropic salts which have been tested and found suitable for use in the present invention include sodium trifluoroacetate, sodium trichloroacetate, sodium perchlorate, guanidine thiocyanate and potassium thiocyanate. In chaotropic salts having an alkali metal cation (e.g., sodium trichloroacetate), substituting a different alkali metal cation (e.g., potassium trichloroacetate) makes little if any difference in chaotropic behaviour.

The concentration of specific chaotropic salt sufficient to effect substantially complete dissolution of a biological source will vary depending on the specific salt and on the nature and concentration of the biological source employed. Typical concentrations of the chaotropic salt are on the order of 5 molar (see Example 3), although other salt concentrations may differ somewhat. For example, a preferred NaI solution is saturated (at 25°C), as hereinafter described. Concentration adjustments represent routine optimization within the scope of the invention. The chaotropic salt must be in a concentration sufficient to effect substantially complete dissolution of the biological source or sample.

The term "biological source" refers to material from any living organism, but excludes purified chemicals (or biochemicals). "Biological source" particularly contemplates biological samples extracted from humans. The inventor has tested the invention using separated cells, pieces of tissue, stool, body fluids (e.g., blood, lymph, urine, saliva, etc.), bacteria, viruses, yeast, and sub-fractions (such as separated nuclei or cytoplasm) of the previous sources with good results. Thus, by the term "biological sample" is intended any and all of the above.

The invention further includes a novel method for selectively separating any specific RNA or DNA from a biological source, said method being herein identified as "homogenous hybridization," "one-phase hybridization," or "liquid-liquid hybridization." By the term "homogeneous hybridization," "one-phase hybridization," or "liquid-liquid hybridization," the terms intended to be synonymous, is meant that both the labeled probe and the target (sample) nucleic acid are in solution during the molecular hybridization reaction between the two, with the molecular hybridization effected in the chaotropic solution.

Regardless of whether RNA or DNA in a nucleic acid-containing biological sample is sought to be separated for identification and/or quantification, the sample is first dissolved in a chaotropic salt solution. If RNA is to be identified and/or quantified, appropriate labeled nucleic acid probe (DNA or RNA) may be added directly to the chaotropic solution of RNA under conditions favorable for hybridization between the labeled probe and the sample RNA.

If DNA is to be measured, the biological sample is dissolved in chaotropic solution and the solution heated prior to hybridization. Heating DNA denatures the molecule, thereby presenting single stranded DNA suitable for hybridization with a suitable labeled probe. In this situation, detergents such as those utilized in the "reverse-probing" described above, are not required.

Typical methods for analytically determining the extent of hybridization (and therefore the amount of RNA or DNA sequence of interest in the sample) as above, is explained in Examples 5 and 10.

Accordingly, the present invention further provides a method for selectively separating a specific RNA sequence from a biological source containing said specific RNA sequence, or determining the presence or absence of said specific RNA sequence in the biological source, comprising the steps of:
A. dissolving said biological source in a chaotropic solution; and
B. contacting said dissolved source, in the chaotropic solution in which said dissolved source is dissolved, with a nucleic acid probe complementary to at least a portion of said RNA sequence, said nucleic acid probe in a soluble form, and incubating the sample thus contacted at a temperature which promotes molecular hybridization and which maintains DNA in an undenatured state.

The present invention further provides a method for selectively detecting and/or quantitating a specific DNA sequence from a biological sample, comprising the steps of:
A. dissolving a biological sample containing said specific DNA sequence by contacting such sample with a chaotropic salt solution;
B. heating the resulting chaotropic solution with sample DNA solubilized therein to at least 45^{o}C to denature said DNA; and
C. contacting said solution containing said denatured sample with a labeled nucleic acid probe complementary to at least a portion of said specific DNA sequence and incubating the thus contacted sample at a temperature promoting molecular hybridization.

The present invention further provides a kit suitable for effecting sample preparation and homogeneous hybridization for evaluating nucleic acids being selected from the group consisting of RNA and DNA, said kit separately comprising:
A. A chaotropic salt;
B. Paraphernalia for conducting molecular hybridization; and
C. An assay for detecting and/or quantitating hybridized duplexes.

The present invention further provides a kit suitable for measuring the amount of specific RNA or DNA sequence, said kit comprising:
A. A chaotropic salt;
B. Paraphernalia for conducting molecular hybridization;
C. An assay for detecting and/or quantitating hybridized duplexes;
D. A probe which comprises a nucleic acid complementary to said specific RNA or DNA sequence; and
E. Positive and negative control biological samples, dissolved in chaotrope.

As discussed above, this homogeneous hybridization provides the particular advantage that the analytical determination of type and/or contact of nucleic acid in a given sample is especially adapted for clinical application. The present invention, combining sample preparation and homogeneous hybridization in substantially the same chaotrope solution, is unique in its ability to retain the accuracy and sensitivity of the most advanced laboratory procedures while possessing the speed, simplicity, economy, versatile and automatability necessary for clinical adaption. For ease of understanding the invention and its novelty, the above parameters are summarized regarding the invention and cross-referenced to pertinent examples of experimentation.

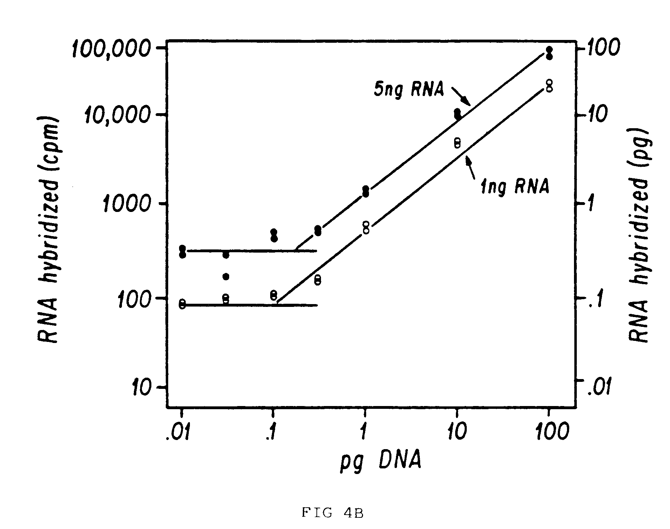

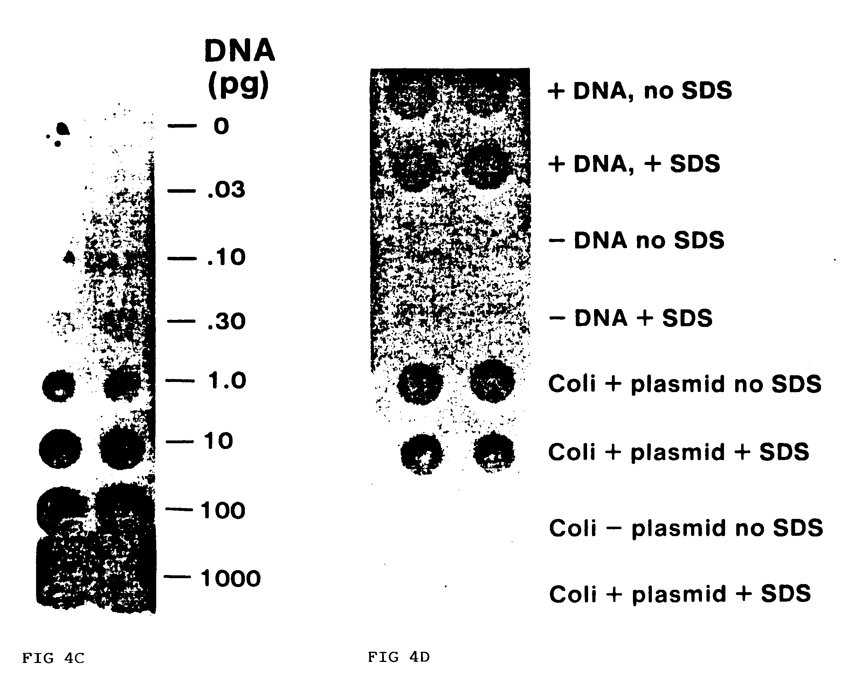

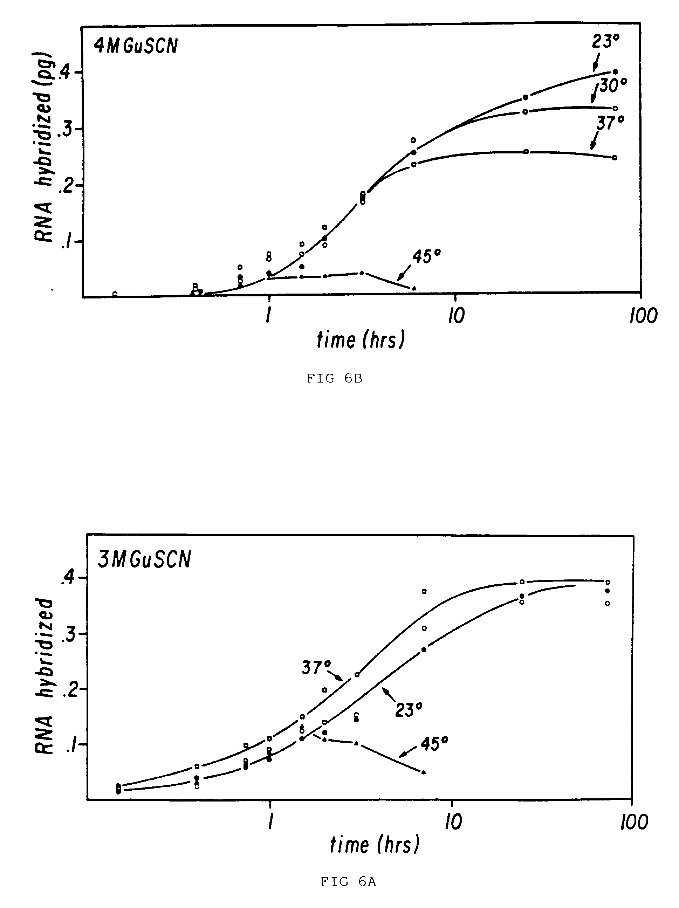

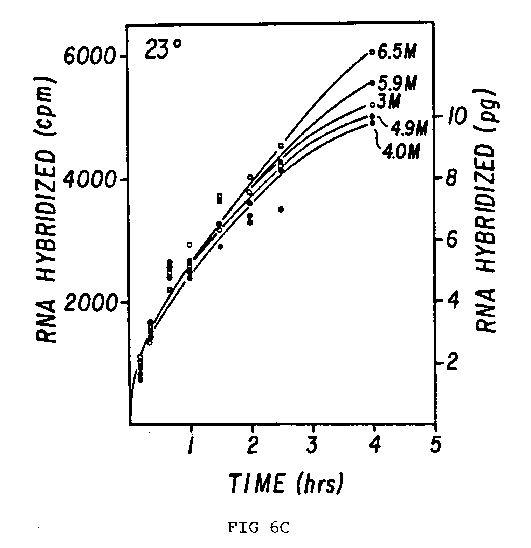

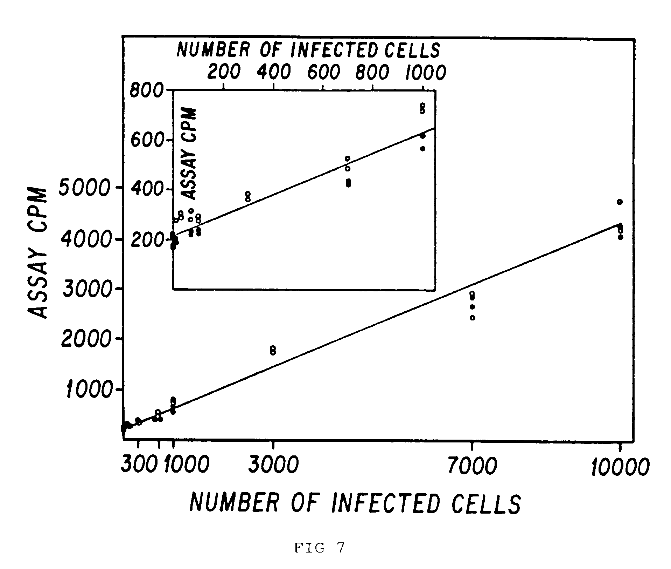

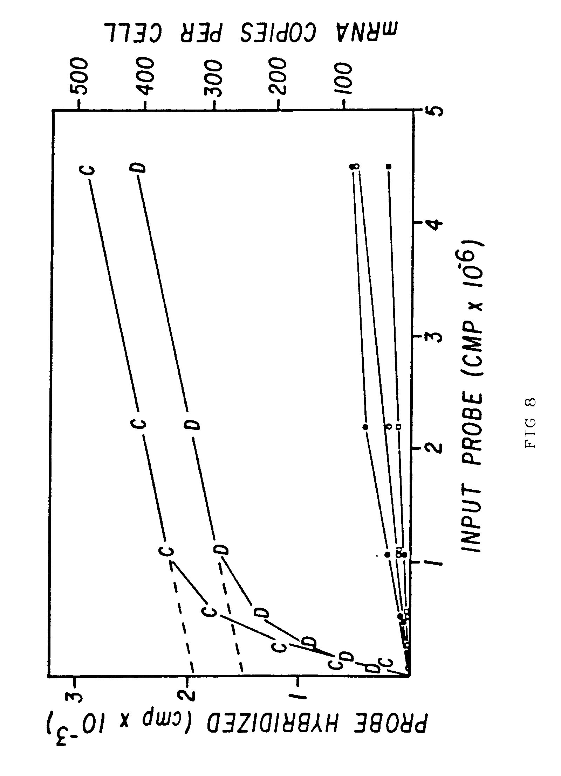

Accuracy - Hybridization in chaotropes operates at 100% efficiency on purified nucleic acids (Example 5, Figure 4 B, upper curve) and operates with equal efficiency on nucleic acids in biological sources (Example 5, Figure 4C). Hybridization in liquid can be described mathematically (Britten and Kohne, Science 161:529, 1968). The present invention performs hybridizations which conform to he mathematical model (Example 7 , Figure 6 ). At probe excess, there is a linear relationship between amount of target nucleic acid present and amount of probe hybridized and the proportionality constant is 1 (Example 5 , Figure 4B, upper curve). With lower amounts of probe, there is still a linear relation between amount of target nucleic acid present and amount of probe hybridized (Example 10 , Figure 7). All of these facts show that the present invention can provide an accurate evaluation of nucleic acids in a biological sample. Example 11 illustrates this in a practical way. (Figure 8).

Sensitivity - Thirty fentograms of complementary target sequence was detected (Example 5 ). This level of sensitivity is state of the art at the moment.

Speed - Regardless of the paucity of target sequence, the invention provides a means for completing sample preparation/hybridization in 15 minutes for target DNA in bacteria (Example 5 , Figure 4D) and in 11 minutes for target DNA in human lymphocytes (Example 5). It follows that target RNA can be evaluated in 6 minutes, since the procedure is identical except that a 5 minute incubation is omitted, but this is not exemplified in the application. It is important to note that full accuracy and sensitivity is retained in these high-speed experiments. One reason the above-mentioned speed is possible is through the unexpected acceleration of hybridization afforded by chaotropes generally (Example 6,, Figure 5A and 5B) and by GuSCN particularly (Example 6 and 7, Figures 5 and 6).

Simplicity - The present invention for evaluating target RNA in a biological sample requires only: 1) contacting a fluid or finely divided tissue with a strong chaotrope for about 1 minute then 2) adding a probe to about 1 ug/ml and incubating the mixture for about 5 minutes. This is exemplified in Examples 10 and 11, except that the hybridization step was prolonged because low amounts of probe were used. It will be obvious to those skilled in the art from Example 5 that had larger amounts of probe been used in Examples 10 and 11, hybridization could have been abbreviated. Further, the probe may be pre-mixed with the chaotrope and the two added simultaneously.

The procedure is simple enough for unskilled persons to perform and, in fact, has been performed in the inventor's laboratory by a junior high school student, a high school student, and a college undergraduate.

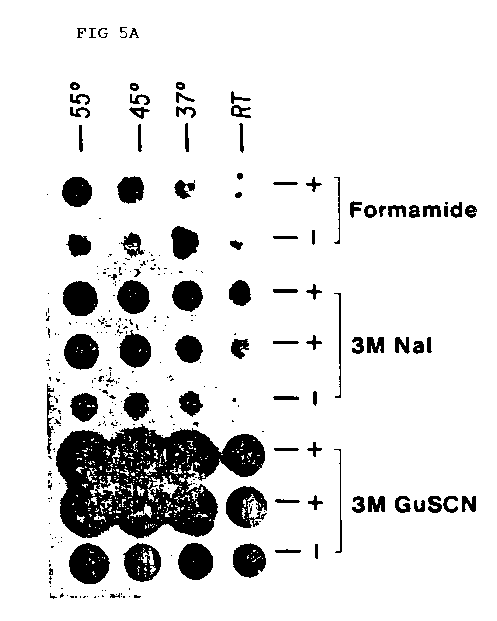

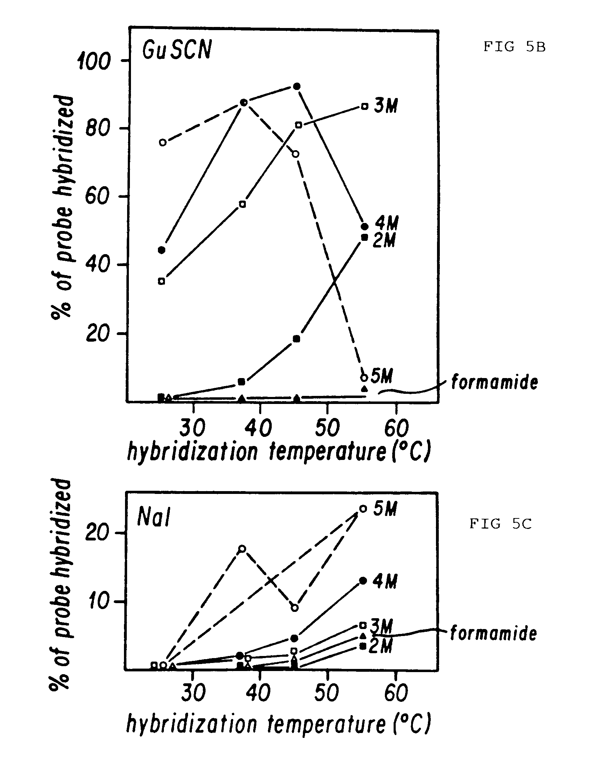

The procedure is not sensitive to perturbations of conditions expected in a clinical laboratory. The process is equally effective from 3-6.5 M GuSCN when conducted at ambient temperature (Example 7, Figure 4C) and using 3-4 M GuSCN is equally efficient from 23-30^{o}C (Example 7 , Figure 6 A, B). Hybridizations can be conducted for 5 minutes or several days (See Example 6, Figures 5A, B and compare with Example 5 , Figure 4D).

That such an extraordinarily simple process would suffice for sample preparation and molecular hybridization was not anticipated by the prior art. Combinations of enzymes, detergents, organic solvents, reducing agents, etc., are usually used during sample preparation. Conditions are ordinarily changed and are quite specific for conducting molecular hybridization. Since target nucleic acids in biological samples are complexed to other macromolecules through many kinds of forces, including ionic bonds, hydrogen bonds and nonpolar bonds, it could not be anticipated that an exposure of a biological sample to a chaotrope would free said target nucleic acids to the extent that they could be probed accurately, efficiently and sensitively. Certainly, it could not be anticipated that such probing could proceed in substantially exactly the same solution used for sample processing. And finally, it could not be anticipated that the rate of the probing process would be accelerated by the chaotrope, providing a faster process.

It should be noted that the simplicity of the chaotrope process was obtained at no sacrifice of accuracy, sensitivity or speed.

Economy - The cost of the chaotrope procedure is lower than all other procedures known to the inventor. Holding such costs as probe costs, hybrid detection costs, sample collection device costs as equal among all systems, the cost of the chaotrope system is the cost of the chaotrope itself, a cost amounting to pennies or fractions thereof per assay. A manual gene diagnosis test using the chaotrope system has been estimated as costing under $1.50 to conduct. The cost of an automated test is not readily calculated, but given the simplicity of the present invention, should be relatively low.

Versatility - Clinical samples are commonly termed "dirty" when compared to research laboratory samples because of the high levels of impurities in such samples as stools, blood, urine, etc. The present invention has been tested across the spectrum of clinical samples and found to operate efficiently in all cases. The inventor is aware neither of prior art achieving this versatility nor of prior art anticipating this versatility. It was quite unexpected to the inventor.

It should be emphasized that, to the best of the inventor's experience, this versatility was obtained with no sacrifice in accuracy, sensitivity, speed, simplicity or economy. Consequently, the present invention unexpectedly embodied the best of all those characteristics which characterize a clinically applicable gene diagnosis process.

Automatability--Until such automation actually exists, it is difficult to argue which of several processes is easiest to automate. It will be obvious to those skilled in the art, however, that the simplicity of the present invention lends itself to automation.

The invention further provides a means for detecting and quantitating HIV nucleic acids in patients.

### BRIEF DESCRIPTION OF THE DRAWINGS

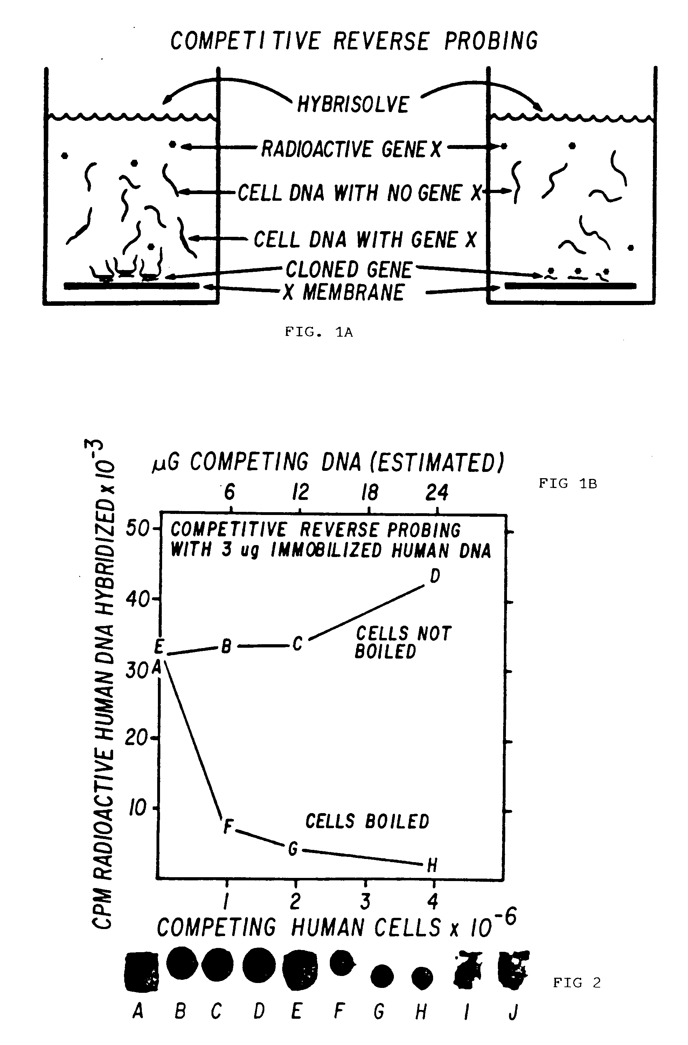

FIGURE 1 is a diagrammatic illustration schematically showing competitive reverse probing;
FIGURE 2 is a graphical illustration of the use of competitive reverse probing in measuring DNA sequences in cells;

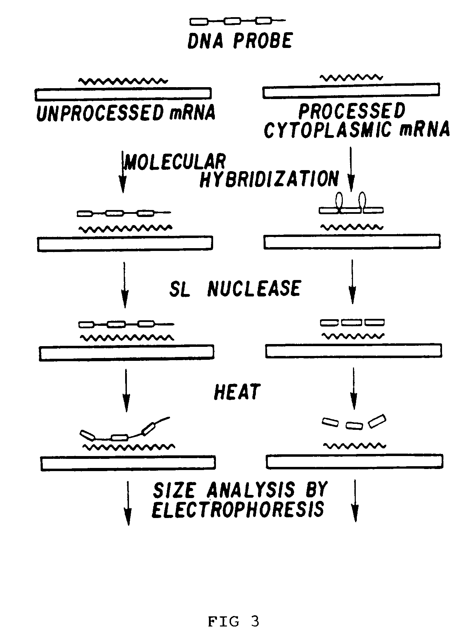

FIGURE 3 is a schematic illustration of a process for determining the structure of an immobilized RNA;

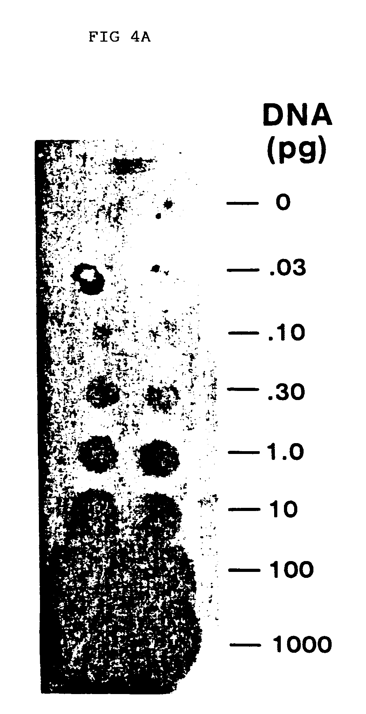

FIGURE 4 represents illustrations of the sensitivity of the invention under various conditions:
   A = pure target DNA, radioautograph
   B = pure target DNA, graphical representation of scintillation counting target
   C = target DNA mixed with cells, radioautograph
   D = target DNA in bacteria, radioautograph
FIGURE 5 represents the efficiency of the invention:
   A = comparison of hybridization in 3M GuSCN, or NaI, or in 50% formamide, radioautograph
   B,C = hybridization of various concentrations of GuSCN or NaI at different temperatures, graphical representation of scintillation counting
FIGURE 6 is a graphical illustration of the kinetics of hybridization in GuSCN:
   A = kinetics in 3M GuSCN at 23, 37, and 45^{o}C
   B = kinetics in 4M GuSCN at 23, 30, 37, and 45^{o}C
   C = kinetics in 3, 4, 4.9, 5.9, 6.5M GuSCN at 23^{o}C;
FIGURE 7 is a graphical illustration of the relationship between the number of virus infected cells dissolved in GuSCN and hybridization values.
FIGURE 8 is a graphical illustration of the relationship between the amount of probe used and hybridization values, demonstrating how to use saturation values to evaluate the number of target RNA molecules present in a biological sample.

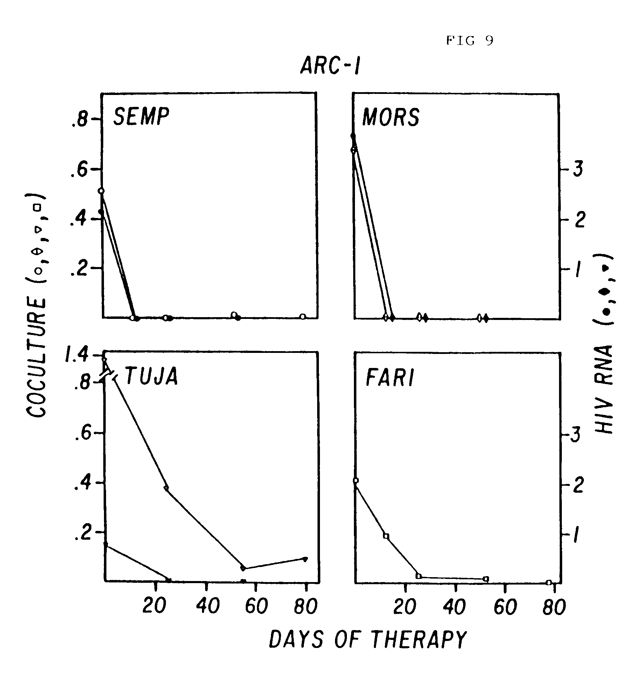

FIGURE 9. Coculture and HIV values obtained on four ARC patients with no detectable serum antigen prior to treatment.

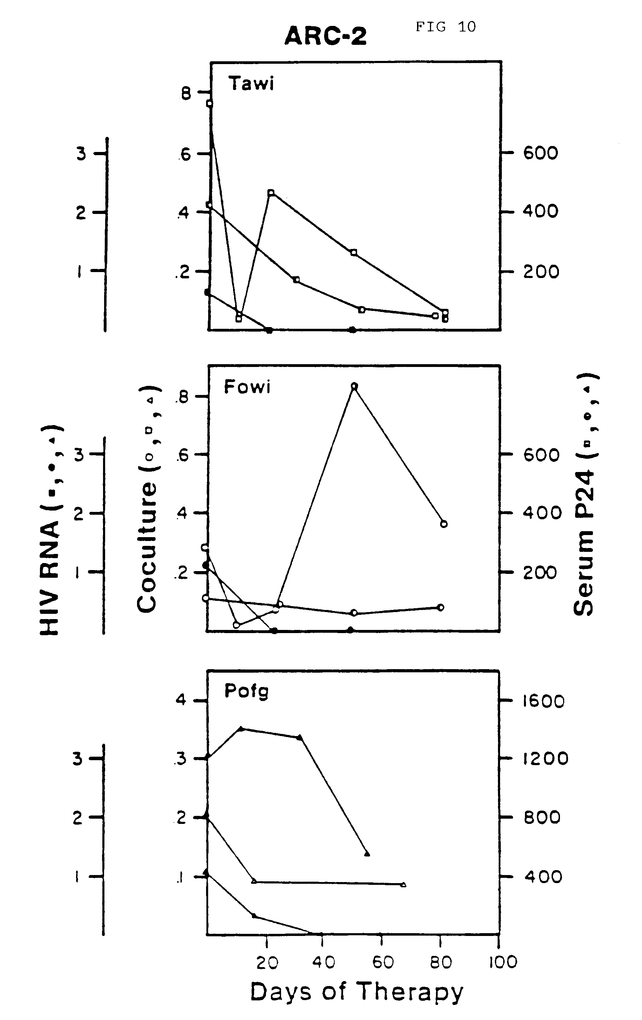

FIGURE 10. Coculture, hybridization, and antigen results obtained with three ARC patients who had detectable serum antigen prior to treatment.

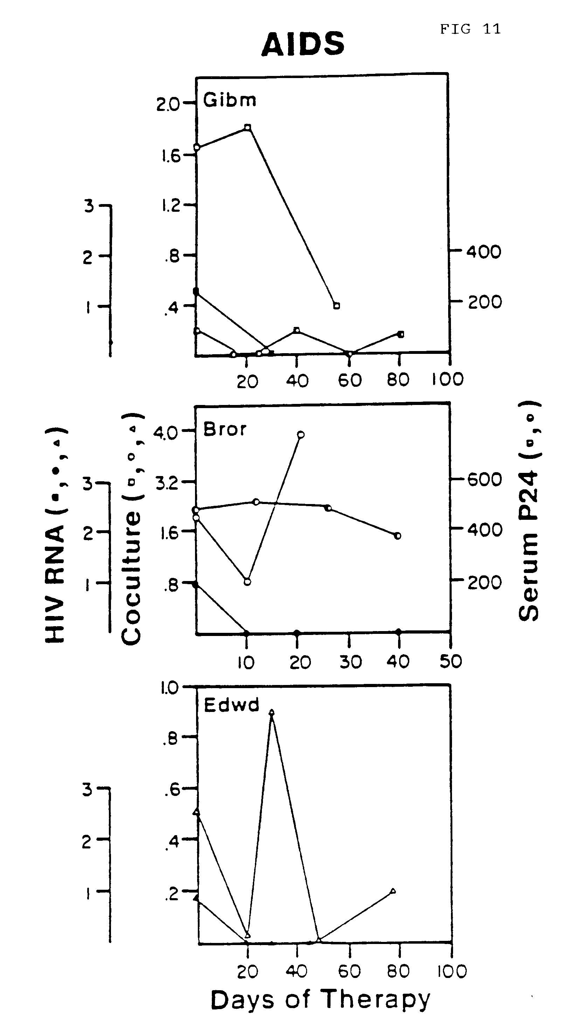

FIGURE 11. Coculture, hybridization, and antigen results obtained with three AIDS patients.

### DETAILED DISCUSSIONS

All temperatures stated herein are Centigrade unless otherwise indicated. "NC" refers to membranes containing nitrocellulose.

### 1. mRNA immobilization procedure for molecular hybridization with auxiliary steps

Message RNA can be selectively immobilized from whole cells according to the following procedure:
(a) prepare biological source
(b) deproteinize enzymatically
(c) add detergents
(d) add chaotropic salt
(e) filter through immobilizing membrane
(f) wash filter (e.g., soak filter in H₂O, EtOH/H₂O, acetic anhydride)
(g) perform molecular hybridization
Of the above steps, (d) and (e) are essential, as previously noted. Steps (a)-(c) and (f) represent auxiliary, non-essential procedures which, depending on the nature of the mRNA source, can be used to enhance the effectiveness of mRNA immobilization as hereinafter explained. Step (g) is the hybridization step which enables quantitation.

### a. Biological Source Preparation

"Preparation" as used herein refers to manipulations required to get the mRNA source in a state suitable for mRNA immobilization while maintaining the primary structural integrity of nucleic acids, insofar as possible. Typical cell preparation manipulations can include removal of fluids from cell or tissue samples, removal of cells or particles from body fluids, preparation of subfractions from cells, fluids, etc. Some examples are illustrated below. Cells can be prepared by any convenient means, providing that care is taken to stabilize mRNA by preventing its structural degradation. Usually, this is accomplished by working with the sample on ice, wearing gloves throughout the procedure, and including cyclohexamide plus ribonuclease inhibitors in solutions which come in contact with cells. Cyclohexamide protects mRNA derived from cells by maintaining the mRNA within a natural structure on ribosomes. Ribonuclease inhibitors reduce the degradation of mRNA by ribonucleases. The ribonuclease inhibitor vanadyl ribonucleosides, prepared by the method of Berger and Birkenmeyer, is satisfactory but remains associated with NC. Vanadyl ribonucleosides will not interfere with molecular hybridization, but they will inhibit reverse transcription and translation of immobilized mRNA. The combination of 0.5 mM aurin tricarboxylic acid (Sigma Chemicals) plus 1 mM hydroxystilbamidine isothamine (Merrell) or 1 U/ml RNAs in (Promega Biotech) do not have this inhibitory action, but they may not be as potent ribonuclease inhibitors as vanadyl ribonucleosides.

If the sample contains mononuclear blood or bone marrow cells, these can be prepared by discontinuous density gradient centrifugation in Ficoll Hypaque. Monolayer cells grown in tissue culture can be released in the usual way with trypsin but this enzymatic treatment does not substitute for the later protease step. Solid tissues may be dissolved directly in a chaotropic salt solution or disassociated to single cells prior to immobilizing. This can be done enzymatically, with DNAase and collagenase (Slocum et al., Cancer Res. 41:1428-1434), but low speed blending or freezing and pulverizing can also be effective. A nearly infinite variety of source preparation steps can be imagined and, to the best of the inventors' knowledge, all of them are compatible with the present invention.

### b. Deproteinization

Even though the bulk of proteinaceous material passes through NC after a biological sample has been dissolved in chaotropic salt solution, enough protein, depending on the nature and amount of the biological sample, may coimmobilize with mRNA so that it is sometimes advisable to degrade as much protein as early in the procedure before filtration as possible. This is conveniently done by adding proteolytic enzymes (called proteases) to prepared cells which are suspended as described above and incubating the cell suspension at 37^{o} for 30 minutes. Proteinase K (Sigma), for example, can be used at about 200 ug/ml. Pronase B (RNAase-free, Calbiochem) is another example of a commercially useful protease, and should be used at 1 mg/ml after a stock 10 mg/ml solution is prepared and incubated for 30 minutes at 37^{o} (Gillespie and Spiegelman, J. Mol. Biol. 12:829-842, 1965). If subcellular fractionation (see below) is desired, the protease step should be delayed.

### Addition of detergents (c) and chaotropic salt (d)

The order of addition of detergents and chaotropic salt to a solution is not critical. Either can be added first, followed by the other, or they can be added together.

Detergents disrupt cells and help suppress protein and DNA immobilization from the chaotropic salt solution.

If the nature of the mRNA source is such that high amounts of protein and/or DNA are available, then addition of a detergent is advisable. Suitable detergents are well known to the art and commercially available. Preferred are non-ionic detergents such as polyoxyethylenes available commercially as the Brij® series (Sigma) or the Tween® series (Sigma). Weakly ionic detergents such a sodium lauryl sarcosinate and sodium desoxycholate also function well. Less preferred but usable if necessary are strongly anionic detergents such as sodium dodecyl sulfate and strongly cationic detergents such as cetyl trimethyl ammonium bromide. Mixtures of detergents can also be employed.

For example, to disrupt cells, add 1/20 vol (note "vol" whenever used herein refers to the sample volume at that point in the procedure) of 10% Brij® 35 (Sigma) and mix the sample. Then add 1/20 vol of 10% sodium desoxycholate (Sigma) and mix the sample again. The amount of detergent which should be added is typically that given above but can depend on the nature of the sample (e.g., blood versus organ tissue) and may be adjusted by means of simple experiments or "trial runs," and is well within the scope of the invention.

Following detergent addition, if needed, add 1 vol of supersaturated chaotropic salt solution to make the cell extract approximately saturated with respect to the chaotropic salt. The strong salt solutions are easily prepared. Using NaI as an example, supersaturated NaI is conveniently prepared by dissolving NaI in hot water (at least 75^{o}C) in a ratio (W/V) of 2.5 gm NaI (Baker) to each ml of hot H₂O. The solution can be stored solid at room temperature, then melted by heating to at least 75^{o} prior to use. A saturated NaI solution is prepared by adding about 1 vol of super-saturated NaI to an NaI-free solution, suspension, biological source, etc. A clear amber solution should result from the addition of 1 vol of supersaturated NaI to suspended cells, tissue sample, or body fluid. Dilutions can be made at this stage into saturated NaI. Similar procedures can be used with the other chaotropic salts, adjusting, of course, for different molecular weights and solubilities, and hence different W/Vs. Making the solution "saturated" in the chaotropic salt is a desirable procedure because of its ease, its reproducibility, and the efficacy of saturated solutions. Lesser concentrations can also be used if desired, however.

### e. Filtration through a nucleic acid-immobilized membrane

Note that the terms "filter" and "membrane" are used interchangeably herein. Most membranes, including nitro-cellulose (NC) and nylon, may be prepared for immobilizing nucleic acids by wetting them in RNAase-free H₂O, then soaking them for 5 minutes or more in RNAase-free 6xSSC. Some hydrophobic membranes may need to be prewet in an alcohol such as ethanol. Membranes can be stored in 6xSSC for several days, at least. In some way, exposure of membranes to a strong NaCl solution activates the membrane for interaction with nucleic acids dissolved in the chaotropic salt solution. Filtration can be performed through dry membranes but considerable lateral diffusion of mRNA occurs, and immobilization may not be quantitative.

Some nucleic acid immobilization procedures (i.e., for mRNA or DNA) will involve filtering several dilutions and/or multiple cell samples. For this reason, manifold devices containing 72-96 wells designed for filtration of large numbers of samples are optimal for the present invention. The Minifold I™, manufactured by Schleicher and Schuell, is particularly suitable because each well has a rather large surface area. The membrane is typically laid on the vacuum chamber of the device over a piece of blotting paper prewet in 6xSSC. The manifold plate is clamped over the membrane and the samples are filtered through the membrane under vacuum.

### f. Washing RNA-containing membranes

The purpose of the washing steps is to remove NaI and non-nucleic acid molecules from the membrane. It is again emphasized that this step is auxiliary, the extent to which it is desirable being determined by the nature of the biological sample being assayed. To remove residual chaotropic salt as well as contaminants in the mRNA source the mRNA filter can be successively soaked in H₂O, 70% ethanol/30% H₂O and acetic anhydride. The mRNA filter can be removed form the manifold assembly and placed directly in a tray containing 1-2 ml of H₂O per cm² of membrane. Reasonable caution should be taken to exclude contact between ribonucleases and the mRNA filter, despite observations that mRNA immobilized on NC in chaotropic salt solution is remarkably resistant to ribonuclease A. Gloves should be worn, the mRNA filter should be handled with clean tweezers and RNAase-free H₂O should be used. The mRNA filter is soaked for at least 5 minutes at room temperature, then the wash solution is changed. Multiple filters can be soaked in a single tray. Filters can be accumulated in the first water wash or, preferably, in the first ethanol wash. Overall, the filter should be soaked in three changes of water, three changes of 70% ethanol/30% H₂O, and once in acetic anhydride.

The acetic anhydride wash can be important for molecular hybridization experiments. Acetic anhydride acetylates basic proteins, minimizing the formation of nonspecific probe-protein complexes and, through a still unknown means, may enhance the molecular hybridization signal. Acetic anhydride is unstable in H₂O so stock solutions cannot be made and stored. The acetic anhydride solution is conveniently made by adding 0.25 ml of pure acetic anhydride (Fisher Chemicals) to 100 ml of 0.1 M triethanolamine (Fisher Chemicals). The solution is vigorously mixed, placed in a clean tray, and the mRNA filter is immediately added. The acetic anhydride soak should be prolonged for 10 minutes at room temperature.

mRNA filters can be removed from acetic anhydride and used immediately or they can be air-dried and stored refrigerated in zip-lock bags. When replicate samples are prepared, it is convenient to number and separate the replicates before storage. Stored filters should be well-dried to discourage microorganism growth. Whether they are used immediately or stored, the mRNA filters are now ready for molecular hybridization.

It should be noted that the concentrations of proteases, ribonuclease inhibitors, and detergents added in auxiliary steps (a), (b), and (c) can be varied depending on the nature of the biological source, and that the values cited herein are intended to be exemplary. Such variation represents routine optimization well within the capabilities of those skilled in the art.

### g. Molecular hybridization of immobilized mRNA

One of the major uses for immobilized DNA or RNA is in determining the quantity of one or a few specific sequences which are present among the total nucleic acid population. Thus, among millions of genes immobilized from a typical mammalian cell, gene probing can detect the presence and determine the quantity of a single gene. And among hundreds to hundreds of thousands of mRNA species immobilized from various kinds of mammalian cells, gene probing can detect the presence and determine the quantity of a single mRNA. This is because, for the most part, each gene and each mRNA species possess a unique nucleotide sequence which can be uniquely and quantitatively recognized by a labeled gene probe through an interactive process called molecular hybridization. Molecular hybridization is a process which is well known in the field of molecular biology for 20 years (see Gillespie, D., and Spiegelman, S., J. Mol. Biol. 12:829-842, 1965; Gillespie, D., Methods Enzymol. 12B:641-668, 1968; Seed, B., Genetic Engineering 4:91-102, 1982; Lehninger, A.L., Biochemistry Text (Worth Publishers), pp. 882-883, 1975; Stryer, L., Biochemistry Text (Freeman and Co.), pp. 600-601, 1975). It involves the formation of hydrogen bonds between two nucleic acids with complementary nucleotide sequences such as is found in the opposite strands of any region of DNA (Watson, J.D., and Crick, F.H.C., Nature 71:737-738, 1953). Thus a labeled probe consisting of one DNA strand or its chemical equivalent (e.g., RNA or modified DNA or RNA of the same nucleotide sequence) can be used to detect and quantitate immobilized DNA or RNA with a complementary or nearly complementary nucleotide sequence.

Molecular hybridization is not, of course, part of the procedure for immobilizing mRNA. Rather, it is a procedural step which allows a specific immobilized mRNA sequence (among many others which would also be immobilized) to be determined. The hybridization is performed by pairing labeled DNA or mRNA (i.e., the probe), which is complementary (i.e., specific) to the mRNA sequence of interest, to the mRNA. Quantitation of the label is directly related to the quantity of the immobilized mRNA sequence of interest. Many labels are possible, such as those falling within the broad categories of radioactive, fluorescent, and enzymatic. For ease of exemplification, hybridization employing radioactive labels will be discussed, but this is not to be taken as limiting.

Many systems have been described for molecular hybridization using radioactive DNA probes. Commonly, the procedure is carried out in three steps: (a) soak the mRNA filter in a solution lacking probe which will minimize interactions between radioactive DNA (the probe) and the membrane ("prehybridization"); (b) incubate the mRNA filter in a solution containing probe which will encourage hybridization between radioactive DNA and mRNA ("hybridization"); and (c) wash away unhybridized probe ("posthybridization"). Commonly used prehybridization solutions which minimize interactions between radioactive DNA and NC generally contain the following ingredients: 0.2% bovine serum albumin (fraction IV, Sigma), 0.2% Ficoll (Type 400, Pharmacia), 0.2% polyvinylpyrollidone (Sigma), 50 ug/ml of low molecular weight DNA (e.g., sonicated salmon sperm DNA, Sigma) and 50 ug/ml of poly (A) (Collaborative Research). Presumably, all of these molecules occupy various sites on the NC which might attract the radioactive probe so that the only possible reactions left will occur during hybridization between the probe and immobilize mRNA. Prehybridization is conveniently accomplished by sealing one or more filters in a seal-a-meal (e.g., Sears) bag with about 1 ml/cm² of NC of a prehybridization solution containing the ingredients listed above. The sealed sack can then be incubated for several hours at the same temperature as will be used for hybridization.

For hybridization, the filter-containing sack is simply opened, drained, replaced with a small volume (0.1-0.2 ml/cm² NC) of hybridization solution and reincubated with gentle shaking. A preferred solution for hybridization of immobilized nucleic acid contains (final concentrations): 50% formamide, pH 7 (Flukka-Granite), 0.45 M NaCl, 0.045 M sodium citrate, 0.05 M sodium phosphate, pH 7.0, 1% SDS (Sigma), and 10⁶-10⁷ cpm/ml of DNA probe. Hybridization is normally conducted overnight at 42⁰ in this solution, though temperatures as low as 20-25^{o} have been successfully used. Higher temperatures provide more specific hybridization.

The nature and amount of formamide are important. Formamide taken directly from the bottle which exhibits a high pH on pH paper will strip immobilized mRNA from its solid support. Similarly, very high concentrations of formamide, even very pure formamide of neutral pH, will remove mRNA from filters. It has not proven to be necessary to redistill or deionize formamide when conducting hybridization, but it is important to test the contents of each bottle periodically for pH and it is wise to pour from the stock formamide bottle, rather than pipette from it. Pure formamide can be stored in dark bottles at room temperature for a small number of weeks; prolonged storage should be at a lower temperature in darkness.

The nature of the DNA probe is also important. Many procedures are now available for the synthesis and purification of DNA probes and new procedures appear continually. The primary criteria for a satisfactory probe are that it be of sufficient chain length to support molecular hybridization (>20 nucleotides) and that it be relatively free of labeled material which will interact with the filter. In terms of probe synthesis, nick-translation can conveniently be accomplished through the use of commercial kits (Amersham) while oligonucleotide-primed copying of gel-purified, denatured DNA still requires individual components (Feinberg and Vogelstein, Anal. Biochem. 132:6-13, 1983). Two useful steps for purification of nick-translated DNA are molecular sieving through Sephadex® G-100 (Pharmacia) followed by filtration through NC (Maniatis et al., Molecular Cloning: A Laboratory Manual, pp. 466-467, 1982). Purified probe is denatured at 100^{o} for 10 minutes, chilled and added last to the hybridization solution.

For posthybridization, filters can be soaked a number of times in 10xSSC plus 0.1% SDS. The time and temperature requirements for these soaks varies with the nature of the probes and cellular samples. Usually three soaks of 30 minutes each at 42⁰ is sufficient to remove unhybridized probe. For quantitation of rare mRNA, it may be necessary to undertake repeated soakings at elevated temperatures for longer times. In any event, these soaks should be followed by a 60 minute soak at 60⁰ in 0.015 M NaCl, 0.0015 M sodium citrate and 1% SDS to remove the last traces of unhybridized probe and to remove probe which has made nonspecific hydrogen bonds with immobilized mRNA. As mentioned above, many systems have been developed for conducting molecular hybridization, including systems containing formamide, urea, ethanol, dimethylsulfoxide, guanidine hydrochloride or high temperatures. All of these systems have been successfully employed with mRNA or DNA immobilized with the present invention.

As will be apparent to one with ordinary skill in the art, procedures similar to those described above, modified appropriately with regard to the particular biological sample and particular RNA being sought (i.e., rRNA from a bacterial source, genomic RNA, mRNA, tRNA and hnRNA) are included within the scope of this invention.

### 11) Molecular hybridization of solubilized sample nucleic acid

A biological sample may be evaluated by a method wherein the biological sample containing 1 or more nucleic acid sequences of interest is prepared by dissolving the sample in a chaotropic salt solution. The nucleic acid sequence of interest (the target nucleic acid sequence) is probed in the chaotropic medium representing the prepared sample utilizing a labeled nucleic acid probe which is complementary to the target nucleic acid sequence.

By the term "evaluated" is intended the detection and/or quantification of target nucleic acid. Accordingly, samples suspected of containing a nucleic acid sequence may be evaluated for the presence or absence of the sequence. Similarly, the sample may also be evaluated by quantifying the amount of target nucleic acid contained in the sample.

Where the sample is being evaluated for the detection of a suspected target nucleic acid sequence, the prepared biological sample may be incubated with a labeled nucleic acid probe containing a nucleic acid sequence which is complementary to the sequence being detected under conditions which will promote hybridization between the target nucleic acid sequence, if present, and the labeled nucleic acid probe. Subsequent to the incubation period, the sample may be tested for the presence or absence of hybridized probe.

Where the sample is to be evaluated in the sense of quantification of the target nucleic acid, quantification of hybridized probe utilizes techniques known to the art.

A collection of various methods for detecting hybridized duplexes can be found in the book "Nucleic Acid Hybridization" (Hames and Higgins, eds.; IRL Press, Washington, D.C., 1985) and in section 1f of "Detailed Discussions," above.

By the term "biological sample" is intended the same material as described above, i.e., separated cells, pieces of tissue, stool, body fluids (e.g., blood, lymph, urine, saliva, etc.), bacteria, viruses, yeast, and subfractions (such as separated nuclei or cytoplasm). See section 1a, "Detailed Discussions," this application.

By the term "solubilizing" is intended that the target nucleic acid be sufficiently separated from other cellular components as to enable the efficient hybridization of said target nucleic acid with labeled nucleic acid complementary thereto while still maintaining the primary structural integrity of the nucleic acids, insofar as possible. See Sections 1a, 1d, "Detailed Discussions," this application.

By the term "contacting" is intended that the biological sample and the chaotropic solution be juxtaposed in such a manner as to permit the dissolution of the sample in the chaotropic solution. Typically, the biological sample is introduced into a container of the chaotropic salt solution.

By the term "chaotropic salt" is intended a salt selected from the group consisting of sodium iodide, sodium perchlorate, potassium iodide, sodium thiocyanate, potassium thiocyanate, guanidine thiocyanate, sodium trichloroacetate, and sodium trifluoroacetate, in concentrations sufficient to achieve the "dissolution" of sample described in the SUMMARY OF THE INVENTION. Other alkali metal salts of the above anions may be used as well. Guanidine thiocyanate is the preferred chaotropic salt.

By the term "nucleic acid probe" is intended any nucleic acid sequence, DNA, or RNA, or modification thereof in labeled form, which will hybridize to at least a portion of the target nucleic acid sequence. The resulting "hybridized duplex" may be a DNA-RNA duplex, a DNA-DNA duplex, or an RNA-RNA duplex.

By the term "complementary" is intended that the target sequence and probe sequence demonstrate sufficient base-pair matching to enable duplex formation under hybridization conditions. It is not required, however, that the base-pair matchings be exact. See section 1f, "Detailed Discussions," this application. Generally speaking, each 10% mismatching between probe and target will retard hybridization rates by a factor of 2 and will lower the Tm of the hybridized duplex by 10^{o}C. (Refer to "nucleic acid hybridization," ibid., pp. 7, 8.)

By the term "conditions which promote molecular hybridization" is intended those conditions known to the art or disclosed in this application for promoting hybridization between two DNA sequences, to RNA sequences, or an RNA and a DNA sequence.

As is known to the art, where one intends to probe for target RNA sequence in the presence of double-stranded DNA sequence, the hybridization conditions must ordinarily be such that the double-stranded DNA sequences remain double-stranded. Similarly, where one intends to probe for a DNA sequence in the presence of RNA sequence, double-stranded DNA must be denatured and then probed under conditions wherein hybridization between probe and any RNA present is avoided or is not detected.

By the term "detecting" is intended both the actual detection and quantification of molecular hybridization. Typical methods known to the art include hydroxyapatite chromatography, enzymic digestion of unpaired probe, membrane filtration, electrophoresis, etc. (refer to "Nucleic Acid Hybridization," ibid., chapters 1-4).

In accordance with this aspect of the invention, the target nucleic acid is solubilized utilizing a chaotropic salt solution as described above and then incubated with a labeled nucleic acid probe. The labeled nucleic acid may be in immobilized or soluble form. The embodiment wherein the labeled nucleic acid probe is in insoluble form is termed "reverse-probing" for the purposes of this invention. Reverse-probing is described below in Example 1.

The embodiment wherein molecular hybridization is achieved under conditions of solution homogeneity, where both the target nucleic acid sequence and the nucleic acid probe are in solution is also introduced in Example 4 and expanded upon in other examples. The invention is typically as follows. A biological sample is first made ready for use. Body fluids are used as is or after fractionation into components such as plasma, cell-free filtrate, etc., which are done by methods which are standard in the art. Cells are made ready by pelleting them from a body fluid or laboratory solution or by suspending such pellets in a laboratory solution, again using standard techniques. Samples of solid tissue are converted to single cell suspension enzymatically or are converted to a suspension or a paste-like consistency by grinding, pulverizing, blending, or homogenizing. These methods are also standard in the art.

The biological sample is then contacted with a chaotropic ion. Guanidine thiocyanate is the preferred chaotropic ion. Typically, the biological source is made approximately 5M in guanidine thiocyanate at room temperature. For solutions or suspensions this is accomplished by adding 0.4 volumes of said solution or suspension to about 1 volume of 7M guanidine thiocyanate and mixing to substantially dissolved solids. For cell pellets or tissue samples converted to a paste-like consistency, a solution of 5 M guanidine thiocyanate is added and the resulting mixture is mixed until solids are substantially dissolved. Other chaotropes than saturated sodium iodide or guanidine thiocyanate have been used to prepare biological samples, such as 6 M sodium trifluoroacetate, 5 M sodium trichloroacetate, and 5 M sodium perchlorate.

As is known in the art, other additives may be added to aid sample dissolution and/or preservation of molecular components as desired. Surfactants including ionic detergents typified by sodium dodecyl sulphate or non-ionic detergents typified by Brij® 35 have been successfully used. The use of detergents and nuclease inhibitors in sodium iodide-containing solutions is known to the art. The use of hydrogen bond breakers and detergents to help dissolve cellular samples is well-known in the art as well.

A novel aspect of the present invention is that the act of dissolving a biological sample in a strong solution of chaotrope renders nucleic acids in said biological source available for probing using the process of molecular hybridization. After the biological source is substantially dissolved, molecular hybridization is achieved simply by adding a gene probe, and incubating the solution or suspension at ambient or a mildly elevated temperature, typically 20-37^{o}C, for a time ranging from a few minutes to several hours. Specific examples of liquid-liquid hybridization are provided below. Alternatively, the probe may be added as a part of the chaotropic solution.

The liquid-liquid hybridization assay of the present invention is suitable for DNA or RNA evaluation of a biological sample. Hybrids were formed between ³²P-labeled RNA probes and nucleic acids in cell lysates. Animal cells or lysozyme-treated bacteria were harvested by centrifugation and dissolved in 5M GuSCN/0.1M EDTA with or without 1M NaCl at a rate of 1 ml of solvent per 10⁷ cells. Cells dissolved readily at room temperature after 2-3 minutes of agitation, yielding a clear moderately viscous, amber solution. Dissolved cells were stored at -70^{o}, except when being thawed to remove aliquots for assays. The solution viscosity decreased after 1-2 freeze thaws.

TARGET DNA. Hybridization of RNA probes with target DNA was accomplished by gently heating the dissolved cells to 60^{o} or above for 5 minutes to denature DNA, adding probe, incubating at room temperature or above and trapping probe RNA: target DNA hybrids on a nitrocellulose membrane as detailed in Example 10. Typically, the equivalent of 10⁵ peripheral blood lymphocytes were dissolved in 10 ul of 5M GuSCN/0.1M EDTA. Two and one-half microliters containing 5 ng of RNA probe diluted in 2xSSC/0.1M EDTA were added and hybridization was accomplished at 25^{o} for 5 minutes. Hybrids were visualized by scintillation counting or radioautography.

RNA. Hybridization of RNA probes with target RNA was accomplished by adding probe to dissolved cells, incubating at room temperature or above, degrading unhybridized probe with RNAase, precipitating hybridized probe with TCA and collecting the precipitate on a nitrocellulose membrane as described in Example 15. Typically, the equivalent of 10⁵ peripheral blood lymphocytee is dissolved in 10 µl of 5M GuSCN/0.1M EDTA. Two and one-half microliters of 2xSSC/0.1M EDTA containing 5 mg of probe is added and hybridization is accomplished at 25^{o} for 5 minutes. Hybrids are visualized by scintillation counting or radioautography.

Regarding handling of data from the liquid-liquid version of the present invention, substantially the same rules apply as were described above in section 4 of "Detailed Discussions," this application.

Several chaotropic salts have been successfully utilized for sample preparation (see Example 3) and two, NaI and GuSCN, have been tested for supporting molecular hybridization (see Example 6 , Figure 5). Both chaotropic salts worked well, giving better molecular hybridization results than the standard systems (formamide and phosphate), but GuSCN was preferred because it unexpectedly accelerated the rate of molecular hybridization over 100-fold, as compared to the NaI system.

### Kits for molecular hybridization of solubilized sample nucleic acid

An exemplary kit for molecular hybridization of solubilized sample nucleic acid according to the present invention can contain at least dark plastic vial of solid GuSCN and solid tetrasodium EDTA to which H₂O or a body fluid can be added to provide a solution of 5M GuSCN/0.1M EDTA. Should H₂O be added, the resulting solution can be added to a biological sample at a rate of 1 ml per 10⁷ cells or the equivalent to provide a solubilized biological sample. Alternatively, a premade solution of chaotrope can be provided. Said kit can also contain a probe for evaluating a given sequence in the biological sample, said probe provided in a quantity to perform about 20 tests (e.g., 100 ng of probe). Said probe can be provided ready to use (e.g., already labeled) or in a precursor form suitable for labeling by the user. The probe may also be a part of the chaotropic solution.

Said kit can also contain "positive control" and "negative control" biological samples dissolved in chaotrope, said biological samples possessing known quantities of specific nucleic acids and results of molecular hybridizations performed on said samples yielding numerical references for quantitating results on unknown test biological samples.

Said kit can also contain materials and devices for hybrid detection, for example filtration solutions, blocking agents, membranes, nuclease solutions, trichloracetic acid, hydroxyapatite, etc.

The present invention further is suitable for detecting and quantitating HIV nucleic acids in patients. One dilemma of evaluating HIV load in ARC-risk, ARC, or AIDS diagnosis is that direct tests of virus antigens or virus infectivity may be difficult or impossible in a percentage of casees because viremia is transient and opposed to varying degrees during the diseases by natural immune mechanisms. This difficulty will be exacerbated if patients are treated with or are induced to develop antiviral antibodies. Moreover, nonproductively infected cells (e.g., harboring latent or defective virus) could contribute to dieases without presenting viral antigens or rescuable infectious centers. However, the sensitivity, speed, versatility, and automatability of the present invention makes such detection and quantitation possible.

Having now generally described the invention, the same will be more fully understood by reference to the following examples which are provided herein for purposes of illustration only and are not intended to be limiting unless otherwise specified.

### Example 1 : Reverse Probing in Chaotropic Salt Solution Using An Immobilized Probe

The term "Reverse Probing" is herein intended to mean a process whereby cells are dissolved directly in a strong solution of a chaotropic salt, heated to denature DNA and destroy DNA or, alternatively not heated, then incubated with a probe-containing membrane under molecular hybridization conditions and under conditions where direct interactions between dissolved nucleic acids and the membrane minimized. This process favors hybridization between the immobilized probe and cellular nucleic acids which are complementary to the immobilized probe. When cells are heated, cellular DNA hybridizes with the immobilized probe; when cells are not heated, cellular RNA hybridizes with the immobilized probe. Reverse Probing utilizes the chemical principles of chaotropic salts which are central to the immobilization process of this invention.

In the foregoing specific embodiments and examples, nucleic acids from cells, viruses, bacteria, etc. were immobilized on a solid support in NaI, then hybridized to a pure radioactive, fluorescent or otherwise tagged probe which was dissolved in an appropriate solution for promoting molecular hybridization. Reverse probing represents a different configuration. In reverse probing, probe can be immobilized onto NC, nylon or another type of membrane, using the present invention or another technique, then hybridization can be carried out by dissolving cells, bacteria, yeast, viruses, etc. or subcellular fractions thereof in a chaotropic salt such as NaI in the presence of additives such as detergents, proteins, oligonucleotides, etc. which reduce direct interaction between DNA or mRNA and the membrane and incubating the resultant mixture with said probe-containing filter. Hybrid formation can be detected by any of several means.

For example, by including in the solution a tagged probe which is complementary to the immobilized probe, hybrid formation between dissolved cellular DNA, RNA and the tagged or immobilized probe (DNA or RNA) can be measured by reduction (competition) in hybridization between the tagged probe and its immobilized complement (Figure 1 ). We call this "Competitive Reverse Probing." Figure 2 presents an experiment to illustrate this principle, showing measurement of human repeated DNA in 10⁶, 2 x 10⁶, or 4 x 10⁶ of blood cells. NC membranes containing 3 micrograms of human repeated DNA were prepared by the present invention. A radioactive probe of human repeated DNA was prepared by nick-translation. Mononuclear blood cells were dissolved in NaI containing 1% Brij® 58 and then duplicate 1 ml aliquots of NaI/Brij® 58 containing 0, 10⁶, x 10⁶ or 4 x 10⁶ cells were prepared. To each aliquot was added 10⁶ cpm of radioactive human DNA (about 10⁻² micrograms). One set of solutions was then boiled for 20 min. to denature DNA. A DNA-containing membrane was added to each solution. Hybridization was effected by incubation for 17 hr. at 37^{o}, then reacted nucleic acids were washed away from the filters. The filters were radioautographed then radioactivity was quantitated by scintillation counting. Both results are presented in Figure 2. It can be seen that in the boiled samples significant competition occurred to the extent that it can be calculated that 10⁶ human cells contains 2-3 micrograms of DNA. Since 10⁶ human cells contains 6 micrograms of DNA of which some 40% is repeated DNA, this result is in excellent agreement with that expected.

Many variation are possible. Specific DNA sequences can be measured by varying the probes. Probes consisting of cloned hepatitis virus DNA permit detection and quantitation of hepatitis virus genomes or mRNA in cells. Immobilization of 1 picogram of probe permits detection of 10⁶ viral genomes. Using other viral probes permits detection of other viral genomes. Eliminating the boiling step and/or preparation of subcellular fractions permits the detection of RNA rather than DNA. In this case, the tagged probe should be single stranded and of the same sequence as the RNA and the probe containing membrane should be pretreated with prehybridization solution. Viral RNAs can be detected and quantitated with this process using viral probes. Cellular RNAs can be detected and quantitated using cloned gene probes such as the myc gene probe but, of course, not limited to that gene probe. DNA or RNA probes can be used. Other membranes that NC can be used as long as 1) probe-membrane complexes can be prepared and 2) hybridization can occur with the immobilized probe in the absence of direct dissolved nucleic-acid membrane interactions during molecular hybridization.

Direct visualization, rather than competitive hybridization is also possible. The experiment described above was repeated, omitting the radioactive probe. Instead of radioautographing the result of molecular hybridization, the membranes were dipped in a solution containing 0.5 µg/ml of ethidium bromide. The filters were then visualized under UV light to detect double stranded nucleic acids on the membrane. No such structures were detected when cells were omitted. Fluorescence was obtained in all cases where cells were boiled, showing directly that hybridization had occurred between the dissolved denatured cellular DNA and the immobilized probe. We call this "Litmus-like Reverse Probing."

All of the variations described above for Competitive Reverse Probing are also possible for Litmuslike Reverse Probing. In addition, many other methods are possible for detecting hybrid structures, including but not limited to, the use of specific antibodies, avidin-biotin complexes, radioactive detection systems, intercalators, etc.

Competitive Reverse Probing and Litmuslike Reverse Probing succeed because of the immobilization principles which are central to this patent application. Chaotropic salts can be used to dissolve cells, denature nucleic acids, prevent nuclease activity and promote specific nucleic acid hybridization. NaI is included in the chaotropic series and is used as the prototype chaotropic salt for the immobilization process. Guanidine thiocyanate has also been used with equal success and presumably other chaotropic salts such as sodium perchlorate, sodium trichloroacetate, sodium trifluoroacetate, etc. will also be useful. Detergents minimize DNA-membrane interactions, a critical aspect of Reverse Probing techniques as it is for the standard immobilization methods. We expect that other features of the present invention, such as selectivity for mRNA will also contribute to the success of the Reverse Probing variation, for example, when using synthetic RNA probes.

### COMPARATIVE EXAMPLE 2:

### Applications of the present immobilization technology

Example 1 dealt primarily with the value of immobilization coupled with molecular hybridization in obtaining precise measurements of the quantity of specific mRNA immobilized from a given number of cells. The present invention also lends itself to procedures for determinations of mRNA structure, for cloning copies of mRNA populations in cells as well as for purifying, analyzing and cloning individual mRNA species. The state of the art with these procedures is outlined below.

### mRNA Structure #1: Modified S1 Nuclease Assay

The structure of an immobilized mRNA can be determined by measuring the size of the probe which has been hybridized to an immobilized mRNA, digested with S1 nuclease, and released from the filter (Figure 3). The experiment has been successfully accomplished by hybridizing long single strands of labeled human DNA to immobilized nuclear RNA of leukemia leukocytes, digesting unhybridized probe to nucleotides with S1 nuclease, releasing S1-resistant probe from the filter and analyzing it by electrophoresis into a polyacrylamide gel.

mRNA immobilization and hybridization was carried out as previously described above. The dot containing the DNA-mRNA hybrid was excised and placed in a solution containing 1000 U/ml of S1 nuclease and was incubated at 45^{o} for 5-10 min. In this experiment undigested probe remained on the filter. Undigested probe can be released from the filter if the S1 nuclease contains ribonuclease or when hybrids are formed with immobilized DNA.

After digestion by S1 the filter dot was removed from the solution, rinsed with cold 0.01xSSPE then plunged for 15 sec. in boiling 0.01xSSPE. The released probe was fractionated by electrophoresis into polyacrylamide and results were displayed by radioautography.

There are no apparent pitfalls in this method which are not inherent in the original Berk-Sharp S1 nuclease assay (Berk and Sharp, Cell 12:721-726).

### mRNA Structure #2: Modified Northern Transfer

In principle, mRNA can be released from one membrane with pure formamide, fractionated by electrophoresis into polyacrylamide then transferred to another membrane for hybridization with a radioactive probe. In practice, mRNA has been released from NC, has been reverse transcribed and translated (Bresser et al., Proc. Nat. Acad. Sci., 1983) and has been reapplied to NC for molecular hybridization (J. Bresser, unpublished observations), but has not yet been successfully fractionated by electrophoresis into agarose. mRNA has been immobilized and released as described above. The released mRNA has been fractionated by electrophoresis into polyacrylamide as described (Bresser et al., Proc. Nat. Acad. Sci., 1983), then the gel was radioautographed. In all instances so far examined the mRNA after release from NC exhibited a lower electrophoretic mobility than prior to immobilization. The reason for this is under investigation.

After electrophoresis, mRNA can be transferred to NC either by Thomas' method (Proc. Nat. Acad. Sci. USA 77:5201-5205) using NaCl or by using NaI (Bresser et al., Proc. Nat. Acad. Sci., 1983). Less convection occurs using NaCl, producing a sharper image on NC. mRNA transferred in NaI is biologically active and can be reverse transcribed or translated.

### Cloning Copies of mRNA Populations in Cells

The construction of "mRNA" libraries from defined cell populations has become an important research tool. Since mRNA immobilized from small numbers of cells can be reverse transcribed into full-length cDNA (Bresser et al., Proc. Nat. Acad. Sci., 1983), a rapid cloning procedure is possible. A problem existed in low efficiency of transcription of immobilized mRNA, but the efficiency of reverse transcription has been increased significantly by (I) eliminating the EtOH and acetic anhydride soaks which can be part of the "mRNA immobilization"; (II) eliminating salt during mRNA-filter prewashes; and (III) using NH₄Ac during precipitation. Second strand synthesis on immobilized mRNA has not been proven, but is indicated from the fact that cDNA is released from the filter when Actinomycin D is not included in the synthesis mixture (J. Bresser, unpublished observations).

### Cloning of Purified mRNA by Immobilization Technology

Cloning of specific mRNAs from different cells provides a reagent for explaining exactly the differences in the regulation of the relevant mRNAs. The outline for this procedure is:
Fractionate mRNA electrophoreticilly in polyacrylamide.
Transfer the mRNA to NC in NaI.
Locate the relevant mRNA species by molecular hybridization.
Melt off the hybridized probe.
Reverse transcribe the relevant mRNA.
Clone the cDNA and screen recombinants.

The method is compatible with any method of mRNA purification. Clearly, the simpler the method of mRNA purification the more powerful the overall method will be. Ideally, the mRNA purification outlined above under "mRNA structure #2: Modified Northern Transfer" will yield suitable mRNA populations for this experiment.

The transfer of mRNA from polyacrylamide to NC must be done in NaI (Bresser et al., Proc. Nat. Acad. Sci., in press, 1983) to preserve biological activity. The procedure for this is as follows: Polyacrylamide gels were soaked in saturated NaI for 30-60 min. The gel was overlaid with a sheet of NC which had been soaked in water, then in 1 M NaCl, then in saturated NaI. The NC was overlaid with absorbent paper towels. Transfer was allowed to proceed until an amount of NaI solution equivalent to ten times the volume of the gel has passed through the gel. The NC was peeled from the polyacrylamide and soaked in H₂O.

Once the total cell mRNA has been transferred to NC the relevant mRNA species can be detected by molecular hybridization. The mRNA-containing band can then be cut precisely from the membrane and the probe can be removed if necessary by dipping the membrane in boiling 0.01xSSPE. Reverse transcription and cloning can follow the procedures outlined in the previous section. Overall, this approach provides an extremely rapid and efficient means of cloning DNA copies of specific mRNAs in instances where mRNA enrichment is useful.

### Example 3: Immobilizations using various chaotropic salts

K562 cells were washed with Hanks salts containing 50 ug/ml of cyclohexamide and 10mM vandayl nucleosides and resuspended at 10⁷ cells/ml in the same buffer. Aliquots of 1 ml were distributed in individual Eppendorf centrifuge tubes and centrifuged at 3000 RPM for 10 min. to pellet the cells. Each individual pellet was suspended in a 5 M salt solution. The salts used were sodium chloride (NaCl), potassium chloride (KCl), potassium bromide (KBr) (B), potassium acetate (KAc), potassium iodide (KI), (GSCN), guanidine hydrochloride (GH), sodium perchlorate (NaClO₄), sodium trifluoroacetate (NaCF₃COO) and sodium trichloroacetate (NaCCl₃COO). Solutions of NaCl, KCl, KAc, and KBr produced turbid nonviscous cell suspensions. Such turbidity is caused by light scattering from undissolved particles (such as whole cells) in the solutions. Therefore, these salts do not dissolve cells. Solutions of KI, NaI, GSCN, NaClO₄, NaCF₃COO and NaCCl₃COO produced clear (nonturbid), slightly viscous solutions representing dissolved cells with freed double-stranded DNA. Heating these solutions to 90^{o}, eliminated the viscosity by denaturing the DNA. Heating the suspensions of 90^{o} caused clumping of cellular material. Thus NaCl, KCl, CAc, GH, and KBr are nonchaotropic salts. The other salts are chaotropic.

One-tenth of one milliliter of each solution was filtered through nitrocellulose and nylon membranes to immobilize DNA. Unheated solutions were filtered at room temperature, 20-22^{o} Centigrade. Heated solutions were filtered while hot, above 50^{o}. Filtration proceeded slowly or not at all in the cases of nonchaotropic salts, and rapidly in the cases of the chaotropic salts. Membranes were washed three times in 20 x SSC (3M NaCl, 0.3 M Na Citrate, then were soaked in prehybridization solution and then hybridized using an abl oncogene probe which was radioactive. After hybridization, the membranes were washed, radioautographed, then individual dots were excised and counted as described in Example 2. Immobilizations of DNA from cells heated in solutions of chaotropic salts and of mRNA from cells in unheated solutions of chaotropic salts produced an intense molecular hybridization signal, while attempted mRNA or DNA immobilization from cells in solutions of nonchaotropic salts gave a much less intense hybridization signal. The reason for this low signal with nonchaotropic salts was several-fold: only part of the sample filtered through the membranes before they became clogged, proteins still attached to the DNA or mRNA hindered immobilization (Bresser, J. Doering, G., and Gillespie, D., DNA 2:243-254, 1983), proteins coimmobilizing and posthybridization steps (Gillespie, D. and Spiegelman, S., J. Mol. Biol. 12:829-842, 1965 compared with Bresser, J., Doering, G. and Gillespie, D., DNA 2:243-254, 1983), etc. This experiment shows that salts of the chaotropic series are useful in the present invention's DNA immobilization, while nonchaotropic salts are not.

The experiment was repeated with a 3:2 mixture of the above-mentioned salt solutions and DMSO. The same nonchaotropic-chaotropic differences noted above without DMSO were also seen with DMSO. No differences were seen comparing results obtained with DMSO to those obtained without DMSO, except that DMSO solutions were less viscous.

The experiment was repeated, including 1% Brij® 58 in all solutions and avoiding any heating above room temperature. These conditions encourage mRNA immobilization rather than DNA immobilization. Cells suspended in nonchaotropic salts plus Brij® 58 remained turbid and nonviscous, probably reflecting intact, undissolved nucleii. Chaotropic salts plus Brij® 58 again produced clear, slightly viscous solutions representing dissolved cells with freed, double-stranded DNA. Cells suspended in nonchaotropic salts plus Brij® 58 filtered slowly or not at all, while cells dissolved in chaotropic salts filtered rapidly. After hybridization to the radioactive abl probe, mRNA immobilized from cells dissolved in chaotropic salts gave an intense molecular hybridization signal, probably for the reasons cited above for DNA immobilization. This experiment shows that all salts of the chaotropic series suffice mRNA immobilization, while nonchaotropic salts do not.

This experiment was repeated with a 3:2 mixture of the above-mentioned salt solutions and DMSO. The same nonchaotropic-chaotropic differences noted above without DMSO were also seen with DMSO. No differences were seen comparing results obtained without DMSO to those obtained with DMSO, except that DMSO solutions were less viscous.

### Example 4:

Chaotropic behavior of various salts was demonstrated using the definition set forth in "SUMMARY OF THE INVENTION." 2x10⁶ K562 human leukemia cells, at a concentration equivalent to 2 mg/ml, were pelleted from culture medium and suspended in 5% glycerol in water as a control and in various salt solutions. The concentration of each salt was 5 molar. Chaotropic behavior was demonstrated if the cells suspended (or dissolved) in the various salt solutions showed a decrease in optical density (read at 600 millimicrons) by a factor of about 2 relative to that shown by the glycerol/water control. Non-chaotropic behavior is demonstrated by little if any change (or a change in the wrong direction, i.e., toward even higher optical density) in optical density. The results are set forth in Table 1.

**Table 1**

| Chaotropicity of Various salts 2 mg cells/ml | | |
|---|---|---|
| Number | Salt | Optical Density⁶⁰ |
| 1 | 5% glycerol in water (control) | 1.26 |
| 2 | culture medium | 1.51 |
| 3 | NaCl | 1.44 |
| 4 | sodium acetate | 1.45 |
| 5 | potassium acetate | 1.46 |
| 6 | potassium bromide | 1.45 |
| 7 | guanidine hydrochloride | 1.42 |
| 8 | NaI | 0.70 |
| 9 | NaClO₄ | 0.65 |
| 10 | KI | 0.70 |
| 11 | NaSCN | 0.46 |
| 12 | KSCN | 0.60 |
| 13 | guanidine SCN | 0.47 |
| 14 | sodium trichloroacetate | 0.34 |
| 15 | sodium trifluoracetate | 0.66 |

Based on the above, it will be readily appreciated that culture medium and salts 3-7 are not chaotropic. Salts 8-15 are chaotropic.

### Example 5 : An Experiment was Conducted to Determine the Speed and Sensitivity of the Invention in Detecting Target DNA Sequences. Target DNA

SP64 plasmid DNA linearized with Eco R1, was diluted into 5 M GuSCN/.1 M EDTA pH 7.0. Aliquots of 10 ul of the DNA dilutions were prepared in duplicate in 500 ul Eppendorf tubes. The tubes were capped, heated to 60^{o} for 5 min., cooled to room temperature and mixed with 5 ul of an RNA probe dissolved in 2xSSC.

### RNA Probe Preparation

The RNA probe was synthesized on supercoiled SP64 DNA, using SP6 RNA polymerase as specified by the manufacturer, promega Biotech. One microgram of DNA and 30 units of RNA polymerase were incubated for 1 hr. at 37^{o}C in 50 µl of a solution specified by Promega Biotech plus nucleoside triphosphates as follows: 500 µM ATP, CTP and UTP plus 5 µM GTP and 200 µC ³²P GTP (3000 C/m mol) . After incubation at 37^{o} DNAase 1 (3 Units from Promega Biotech) was added and incubation was continued at 37^{o}C for 15 min. Diethyloxydiformate (5 µl; Eastman Chemical; also known as diethylpyrocarbonate) was added and the emulsion was vigorously agitated for 5 sec. The volume was adjusted to 200 µl with TE buffer and unreacted nucleotides were removed by spun chromatography through Sephadex G50 as outlined by Maniatis et al., Molecular Cloning, published by Cold Spring Harbor (1982). The flow-through was made 0.4 M in NaCl, heated 20 min to 100^{o} and filtered through 2 layers of nitrocellulose (BA85, Schleicher and Schuell). The filtrate was diluted to 10⁶ cpm/µl with 2xSSC and stored at -20^{o}. For long-term storage the probe was precipitated from 2 vols of ethanol, collected by centrifugation at 12,000 x g for 15 min, dissolved in 100% formamide and kept at -20^{o}. The probe was diluted fivefold in 2xSSC before use (concentration = 2·10⁵ dpm/µl; ca. 2 ng/µl or 5 ng/hybridization reaction). The probe represented 600 nt of the 3,000 nt SP64 DNA.

### Molecular Hybridization

Immediately after the probe was added, the solution was transferred to a 37^{o} bath and held there for 2 hr to allow molecular hybridization between the RNA probe and the SP64 target DNA.

### Hybrid Detection

After hybridization, 200 µl of 2xSSC/.1 M EDTA pH7/50 µg per ml of polyadenylic acid were mixed in and the resultant solution was filtered through nitrocellulose (BA85, Schleicher and Schuell) at a rate of about 1 ml/min. The nitrocellulose had been wet in H₂O and soaked briefly in 2xSSC prior to filtration. The bulk of unreacted RNA probe flowed through the membrane while DNA and associated RNA probe became membrane-bound.

After filtration the membrane was soaked for 30 min at 55^{o} in 50 ml of 2xSSC/20 ug per ml of ribonuclease A/20 units per ml of ribonuclease T1. This step removes adventitiously bound probe, leaving only hybridized probe associated with the membrane. Radioactivity on the membrane was estimated by radioautography with x-ray film (Figure 4, panel A), and quantified by scintillation counting (Figure 4, panel B).

As Figure 4, panels A and B show, the amount of RNA probe associated with the membrane was a direct function of the amount of target DNA. The smallest amount of DNA giving an increased radioactivity signal ("positive hybridization") in this experiment was 0.3 picograms of DNA. This corresponds to the detection of about 300,000 gene-size DNA molecules (ca. 1000 nucleotides long), in a 3 hr assay. These speed and sensitivity parameters are within the requirements of most situations where gene diagnosis will be initially used. Since the probe only comprised 1/5 the complexity (length) of the target and since only one of the two target strands is detected, .03 pg (30 femtograms) of complementary target sequence was measured in this experiment).

Panel B is a quantitative represented of results like those illustrated in Panel A. Note that with 5 ng of probe, the quantity of probe hybridized equals exactly the quantity of complementary target DNA present; that is, hybridization is 100% efficient with regard to saturating target sites.

The experiment was repeated in a setting more comparable to gene diagnosis. Cells were first dissolved in 5M GuSCN/.1 M EDTA pH 7.0 at a rate of 10⁷ cells/ml. This was done as follows: Five milliliters of blood were drawn into tubes containing heparin. Mononuclear cells were prepared by centrifugation into Ficoll-Hypaque using methods which are standard in the art. The mononuclear cells were diluted with PBS, counted in a hemocytometer, pelleted by centrifugation and 1 ml of 5·M GuSCN/.1 M EDTA pH 7.0 was added for each 10⁷ cells pelleted. The cells substantially dissolved after 1-2 min of gentle agitation.

SP64 plasmid DNA linearized with Eco R1 was diluted into the solution of dissolved cells. Aliquots of 10 ul of the DNA dilutions were prepared in duplicate in 500 ul Eppendorf tubes. The tubes were capped, heated to 60^{o} for 5 min, cooled to room temperature and immediately mixed with 5 ul of the RNA probe described above. Incubation at 37^{o}, dilution into 2xSSC/.1 M EDTA pH7.0/50 µg per ml of polyadenylic acid, filtration, nuclease treatment, filming and scintillation counting were performed as described above. As can be seen in Panel C of Figure 4, substantially the same sensitivity of target DNA quantitation was achieved in the presence of dissolved cells as in their absence. This result established that the present invention can be successfully used for gene diagnosis on a clinical sample.

The experiment was repeated to learn whether the invention could be used on a body fluid, rather than a single-cell suspension. First, a solution of 7 M GuSCN/.14 M EDTA was prepared by mixing 16.6 gms of solid GuSCN (Flukka Chemicals) with 5.6 ml of 0.5 M EDTA. The volume was adjusted to 25 ml and solids were dissolved by gentle heating. One-half milliliter aliquots were dispensed while the solution was warm. The solution solidified upon cooling to room temperature.

Two-tenths of a millilter of whole, heparinized blood were added to one 0.5 aliquot of 7 MGuSCN/.14 M EDTA and the system was gently shaken at room temperature until solids were substantially dissolved, typically 1-2 min.

SP64 plasmid DNA linearized with Eco R1 was diluted into the solution of dissolved blood and probed as described above, except that 1) the probe was prepared in 5 M GuSCN so that the system was maintained at 5 M GuSCN during hybridization, 2) hybridization was done at 23^{o} and 3) hybrids were diluted into 200 µl of 2xSSC/.1 M EDTA, pH 7/50 µg/ml of polyadenylic acid and filtered through BA85 NC as above. Substantially the same sensitivity of target DNA quantitation was achieved in the presence of dissolved blood with dissolved cells or pure GuSCN/EDTA solution.

The experiment was repeated to learn whether the invention could be used on a solid tissue. A biopsy of lung cancer was obtained as a leftover piece from a pathology study. The tissue specimen was weighed and then was laid upon a stainless steel box filled with liquid nitrogen. After the tissue had frozen, it was pulverized with a pestle cooled in liquid nitrogen. The powder was transferred into a tube and 5M GuSCN/.1 M EDTA pH7 was added at a rate of 1 ml per 10 mg of powder. After gentle agitation for 1-2 min the powder was substantially dissolved.

SP64 DNA linearized with Eco R1 was diluted into the solution of dissolved tissue and probed as described for 5 M GuSCN lacking tissue. Substantially the same sensitivity of target DNA quantitation was achieved in the presence of dissolved tissue as with dissolved blood or dissolved cells or with pure GuSCN/EDTA solution (data not shown).

The experiment was repeated to learn whether the invention could be used on intact bacteria. E. coli carrying the SP64 plasmid was incubated with 2 mg/ml of lysozyme at 25^{o} for 5 minutes, then was made 5M GuSCN/.1M EDTA as described above for whole blood and heated 5 minutes at 60^{o}. Hybrids were formed in 15 µl 3M GuSCN/.06M EDTA with 10 mg of RNA probe. Hybridization was for 5 minutes at 25^{o} Results were determined radioautographically (Figure 4D) and by scintillation counting. The sensitivity of detection of intracellular plasmid was compared with that of an equivalent amount of purified, linearized DNA. the detection of intracellular plasmid (2569 cpm hybridized) was about half as efficient as the detection of purified DNA (5177 cpm hybridized). The inefficiency probably resulted from incomplete hybrid capture on nitrocellulose, since other detection methods, using oligodeoxynucleotide probes have consistently shown measurements of intracellular plasmid to be 90-115% as effective as marked DNA detection (M. Collins, personal communication). SDS was included in some samples to aid bacterial lysis but was found to be unnecessary and, in fact, hindered hybrid immobilization. Incubation at 105^{o} can be used in place of the lysozyme pretreatment.

The above experiment illustrates the speed with which the invention can be employed. This speed is also illustrated by the experiment described below.

The experiment was repeated to learn whether the invention could be used on intact cells to measure HIV virus RNA. One hundred thousand infected or uninfected lymphocytes were dissolved in 10 µl of 5M GuSCN/.1M EDTA/1M NaCl and heated to 60^{o}C for 5 minutes. Hybridization of the dissolved cells with 10 ng of an RNA probe corresponding to the Pstl-EcoRi gagpol fragment of HIV virus was conducted in 12.5 µl of 4M GuSCN/.08M EDTA/.8M NaCl for 5 minutes at 25^{o}C. Hybridization was quantitated by scintillation counting. Results were as follows: Culture C (Infected) = 5555 cpm hybridized, Culture D (infected) = 3585 cpm hybridized, Culture E (uninfected) = 1031 cpm hybridized and Culture F (uninfected) = 1044 cpm hybridized. Since other experiments showed the reaction to be kinetically complete, since the probe had a specific activity of 10³ cpm/pg and since the complexity of the target was 1500 nucleotides, the result shows an average of 13 copies per cell of virus genes in culture C and 7.5 copies per cell of virus genes in culture D. Thus, this experiment illustrates the speed, simplicity and quantitative nature of the invention.

Overall, this Example illustrates several advantageous features of the invention. In addition to speed, simplicity and quantitation as mentioned above, the experiments of this Example demonstrate the versatility of the invention when employed on a wide variety of clinical samples.

It will moreover be obvious to those skilled in the art that the relatively small number of manipulations and the mild temperatures employed mean that the invention is well suited to automation.

All of these features combine synergistically to provide a clinical applicability not anticipated in the prior art.

It should be noted that the practice of the invention is independent of the nature of the probe and the means of hybrid detection. For example, probes complementary to a different fragment of the viral genome may be used. Further, double-stranded probes or oligonucleotide probes have been used instead of single-stranded probes with similar or equal efficacy. Probes labeled with biotin have been used, and others labeled with enzymes, metals or antigens might have been used instead of a radioactive probe. Photon emission resulting from an energy transfer process or some other method might have been used instead of filtration to measure the extent of hybridization. The essence of the invention with respect to this Example 5 is the efficient solubilization of DNA from a biological sample and its denaturation essentially immediately permitting its hybridization with a DNA or RNA probe.

### Example 6 : An experiment was conducted to show the efficiency of liquid-liquid hybridization in GuSCN, NaI and formamide.

An RNA probe was prepared as described in Example 5 . Twenty-five pg of RNA probe was hybridized with linearized, denatured SP64 DNA (see Example 5 ) in 15 µl of hybridization solution. 1 Hybridization solutions consisted of various concentrations of GuSCN or NaI in H₂O (2M, 3M, 4M or 5M) or 50% formamide, .9m NaCl/.09M Nacitrate/.014M NaPhosphate, pH 6.8. Hybridization was conducted for 2 hours at 25, 37, 45 or 55^{o}.

To measure hybridization with target DNA excess, RNA-DNA hybrids formed in 15 µl with 200 ng of DNA target were filtered as described in Example 10 through nitrocellulose and radioautographed. In 5M GuSCN lacking EDTA, in 2-3m NaI or in 50% formamide/0.9M NaCl hybridization of a trace of RNA probe (25 pg/15 µl) to an excess of DNA target (200 ng/15 µl) proceeded efficiently, under optimal conditions converting 90% of RNA chains to RNA-DNA hybrid structures. Routinely, 40-50% of input radioactivity could be converted to hybrids which resisted RNAase treatment in 0.3M NaCl at 23^{o}.

In experiments with only a modest excess of target DNA (10 ng in 15 µl) the extent of hybridization of 25 pg of RNA probe proceeded less efficiently in 2 hours at suboptimal conditions, but at the Tₒₚₜ in 3-5M GuSCN over 90% of probe RNA chains were captured in hybrid complexes with DNA (Fig. 5 A) . The hybridization efficiency in GuSCN was 50-100 times that observed in 50% formamide/0.9M NaCl at 42^{o}C in parallel experiments (Figure 5 B). Hybridizations in concentrated NaI proceeded inefficiently (Figure 5 C).

Optimal conditions varied with varying GuSCN concentration (Figure 5) lower optimal temperatures were correlated with higher GuSCN concentrations as expected. The temperature optimum in 3M GuSCN was over 55^{o} while in 5M GuSCN the Tₒₚₜ was around 37^{o}. Nevertheless, even with low quantities of probe, the hybridization reaction approached completion in 2 hours over a wide range of conditions (Fig. 5 B).

### Example 7 : Experiments were conducted to determine the speed of molecular hybridization in GuSCN

An RNA probe was prepared as described in Example 5 . RNA probe was hybridized with linearized, denatured SP64 DNA (see Example 5 ) in 15 µl of hybridization solution. Hybridization solution consisted of various concentrations of GuSCN in 0.1M EDTA, pH 7.0. Hybridizations were conducted at various temperatures for various lengths of time.

Based on direct comparisons with hybrids formed at 37^{o} in 50% formamide in probe excess (e.g., Example 6 ), hybridization in GuSCN was accelerated some 100-fold. Similarly rapid kinetics were obtained at lower probe concentrations (Figure 6A and 6B). Using 1.3 ng/ml of probe, and 0.25 ng/ml of complementary target DNA hybridizations in 3 or 4 M GuSCN were essentially over in 5-10 hours. The time of half-completion was about 3 hours, corresponding to a Cₒt1/2 of .03 x 10⁻³ instead of the 2x 10⁻³ expected for nucleic acids of complexity 1 kb. This amounts to a 70-fold calculated acceleration over Britten and Kohne standard conditions (Science 161:529, 1968). No further acceleration was obtained with 1M NaCl. The hybridization rate was essentially constant at 23^{o} over a wide range of GuSCN concentrations (Figure 6 ).

High concentrations of chaotropes during hybridization lower the hybridization Tₒₚₜ and accelerate the hybridization reaction. Tₒₚₜ values will probably vary with each chaotrope as specified by Hamaguchi and Geiduschek (J. Am. Chem. Soc. 84:1329, 1962). In practice, GuSCN was preferred over NaI because the hybridization efficiency with GuSCN was always superior and because the hybridization rate accelerations were greater.

RNA-DNA hybridizations can be performed at room temperature in 3-6M GuSCN. The t_{1/2} for hybridizations using nanograms of RNA and complementary target DNA per milliliter is about 4 hours. Hybridizations driven with 100 ng of probe per milliliter are 75% or more complete in 5 minutes at room temperature.

### Example 8: Use of the Invention for Virus DNA Diagnosis on Whole Blood Samples

Blood was collected into evacuated tubes containing heparin from two individuals with active hepatitis and hepatitis B virus DNA sequences in mononuclear cells as well as from two normal, virus-free volunteers. Five microliters of whole unclotted blood were mixed in a conical-bottom 0.5 ml plastic tube with five microliters of liquified supersaturated NaI. Five microliters of a solution containing 10 ng tricine, pH 7, 0/ .1 mM dithiotreitol and 100 ng of a single-stranded, ³²P-labeled, RNA probe consisting of the whole Hepatitis B virus genome was added and the solution was thoroughly mixed. The probe was prepared as specified in Example 5 but using a recombinant SP64 DNA template into which had been cloned the whole Hepatitis B virus genome. The solution was incubated at 90^{o} for 5 minutes to denature sample DNA. Some tubes were transferred to a 37^{o} water bath and incubated there for 4 hrs to permit molecular hybridization. After hybridization, the solution was expelled into a tube containing 350 microliters of 2xSSC/.1 M EDTA/50 ug/ml of poly (A). Hybridized probe molecules were trapped on a nitrocellulose membrane by filtration under mild vacuum (see Example 5). Some membranes were incubated for 30 min at 37^{o} in 0.4 M NaCl/0.05 M tris, pH 7.2/20 g per ml RNAase A to destroy residual, unhybridized RNA probe. All membranes were soaked for 15 min in 0.4 M NaCl/0.05 M tris, pH 7 at room temperature. The extent of hybridization was assessed by radioautography using x-ray film and quantitated by analyzing radioactivity on the membrane in a scintillation counter using conventional techniques. As can be seen in Table 5 a greater hybridization signal was obtained with blood from individuals with hepatitis, compared to the unafflicted controls, as long as the 4 hr incubation to promote molecular hybridization was included.

**Table 2**

| Source of Blood | no INC 37^{o} | | 4 hr 37^{o} | |
|---|---|---|---|---|
| | - RNAase | +RNAase | - RNAase | +RNAase |
| normal #1 | 286 | 45 | 416 | 39 |
| normal #2 | 203 | 34 | 387 | 47 |
| Hepatitis #1 | 277 | 67 | 1098 | 386 |
| Hepatitis #2 | 308 | 29 | 1469 | 593 |

Detection of viral DNA sequences in blood of hepatitis patients, using the invention. Each number is the average of three determinations, measured by scintillation counting.

Alternatively, a single-stranded DNA probe consisting of the whole hepatitis virus genome was substituted for the RNA probe and the hybridization procedure was carried out as described above. After molecular hybridization the sample was diluted into 1 ml of a solution containing 0.05 M tris-HCl, pH 7.0/0.4 M NaCl/0.1 M ZnCl₂/10 U of Sl nuclease and incubated at 45 for 30 min to destroy unhybridized probe as described by the manufacturer, Worthington Biochemicals. Hybridized probe was collected by denaturation and filtration through nitrocellulose (Nygaard and Hall, 1964).

In all of these experimental situations, hybrid formation using blood from the patients with acute active hepatitis and hepatitis virus DNA sequences in mononuclear blood cells was compared with hybrid formation using blood obtained from the virus-negative, normal controls. In every experimental situation, more membrane-bound radioactivity (e.g., molecular hybridization) was obtained with blood from the individuals with hepatitis.

### Example 9 : Use of the Invention for Bacterial DNA Diagnosis on Stool Samples

A stool sample was obtained from a child with diarrhea. To an aliquot was added the bacterium, Campylobacter, at a rate of 10⁴ organisms per milliliter of stool. Stool sample was added to 0.5 gm GuSCN to give 1 ml of solution at 5 M GuSCN. The sample was incubated at 105^{o} for 5 minutes.

Ten microliters of the stool sample was thoroughly mixed with 2.5 ul of a solution containing 10 ng of a single-stranded, ³²P-labeled RNA probe consisting of a transcript of 10 Kb of the Campylobacter genome. The samples were incubated at 23^{o} for 30 min to allow molecular hybridization and hybrids were detected as described in Example 5 . The stool aliquot to which Campylobacter had been added yielded significantly more molecular hybridization than the stool aliquot lacking Campylobacter.

### Example 10: Use of the invention for detecting HIV Viral RNA in small numbers of cells

Uninfected and HIV infected cells were dissolved in 5M GuSCN/.1M EDTA at a rate of 10⁷ cells/ml. HIV infected cells were serially diluted into 5M GuSCN/.1M EDTA or into the uninfected cells dissolved in 5M GuSCN/.1M EDTA. To 10 ul aliquots, 2.5 µl containing 100 pg of the gag-pol RNA probe described in Example 5 was added. Hybridization was conducted at 25^{o} for 48 hours.

After hybridization, 200 µl 2xSSC/.1M EDTA/10 µg Poly(A)/4 µg RNAase A/4U RNAase Tl were added and unhybridized probe was digested during a 30 minute incubation at 25^{o}. The solution was chilled, made 10% in TCA and hybridized probe was collected on a nitrocellulose membrane using procedures which are standard in the art. Radioactivity was determined by scintillation counting.

It can be seen from Figure 7 that the quantity of probe hybridized was a linear function of the number of infected cells from 100 to 10,000 cells, whether they were diluted into 5M GuSCN or into infected cells dissolved in 5M GuSCN. As few as 10 cells gave positive hybridization values.

### Example 11: Use of the invention to quantify HIV viral RNA in cells

HIV-infected cells were dissolved in 5M GuSCN/.1M EDTA at a rate of 10⁷ cells/ml. To ten microliters of cells was added 5 ul of a solution containing various amounts of the gag-pol RNA probe described in Example 5 . Hybridization was conducted at 25^{o} for 17 hours. Hybrids were processed as described in Example 10.

It can be seen from Figure 8 that hybridization using infected cells increased rapidly with increasing probe then "plateaued," thereafter increasing at essentially the same rate as when using uninfected cells. The maximum amount of hybridization, less the plateau is the "saturation" value, i.e., the value of amount of probe hybridized when all target RNA sites are occupied. Since at probe excess, the efficiency of saturating target sites in 3M GuSCN in 100% (Fiqure 4B of Example 5), the ug of probe hybridized equals the ug of target RNA present in the sample. Therefore, the results of Figure 7 show 200 and 150 pg of target RNA per 10⁵ cells of culture C and D, respectively. Since the probe has a complexity of 1.5 nucleotides, there are 3,250 and 3,000 molecules of viral RNA per cell in culture C and D, respectively.

This example illustrates the power of the invention for quantifying the number of target RNA molecules in a clinical sample.

### Example 12: Measurement of HIV Nucleic Acids in Patients

The present invention was performed on a series of 10 patients with ARC and AIDS who were treated with Ampligen. This mismatched dsRNA molecule is both an antiviral and an immune enhancer and, in particular, a potent inhibitor of HIV infection in vitro. Before therapy, all 10 patients in this study demonstrated circulating antibodies against HIV which reacted with the major viral proteins including p24. Additionally, the 10 patients were HIV-positive by the coculture assay on two or three occasions preceding Ampligen treatment.

The number of RNA molecules per 250,000 cells was measured by molecular hybridization with a ³²P RNA probe complementary to the 5' end of the gag gene and most of the pol gene of HIV (see Example 5 ) . Mononuclear cells purified from heparinized blood by Ficoll gradient centrifugation were then harvested by centrifugation and dissolved in 5M GuSCN/.1M EDTA/10 mM DTT at a concentration of 10⁷ cell/ml (see Example 5 ). Under these conditions, lymphocytes essentially dissolve and target RNA is liberated in a form directly suitable for efficient molecular hybridization. RNA probe was added directly to GuSCN-dissolved cells and molecular hybridization was conducted at 26^{o}C for 44 hours in 4M GuSCN. RNA-RNA hybrids were purified by the RNAase/TCA method described in Example 10.

Figures 9 to 11 are graphical illustrations depicting changes caused by chemotherapy in AIDS virus RNA present in blood cells of ten patients with AIDS or ARC (AIDS-related complex), using the present invention. Results are contrasted with coculture and direct serum antigen measurements.

Hybridizations were performed with 25 pg of probe and 250,000 cells in 4M GuSCN at 26^{o}C. Hybridization values were converted to number of target RNA molecules after subtracting negative controls. Negative controls equaled 0.1-0.2% of input probe. One cpm = 1500-3600 HIV RNA molecules, depending on the specific activity of the probe.

Prior to Ampligen therapy, HIV RNA could be detected in blood cells of nine of the ten patients by direct molecular hybridization (filled symbols on ordinate, Figures 14-16). The tenth patient (Fari, Fig. 9 ) appeared to be hybridization-negative after subtraction of an unusually high negative control value and may have been a false negative for this reason. HIV RNA values were generally around 100,000 molecules per 250,000 mononuclear blood cells, corresponding to about one infected cell in 10⁴ blood mononuclear cells. Since hybridizations were conducted with low amounts of probe, these are minimum HIV RNA values. Serum HIV antigen was detected in only 5 of the 10 patients prior to treatment (Gibm, Bror, Edwd, Tawi, Fowi) (half-filled symbols of Figures 9 to 11).

HIV RNA in circulating blood cells measured by molecular hydridization became undetectable in all patients at the first time point taken after beginning Ampligen therapy. Two apparently positive hybridization results at eight weeks were associated with unusually low negative controls and proved to be negative on retesting. The present example shows that HIV RNA in circulating blood cells may be directly quantitated in the presence of a chaotropic solution by use of direct molecular hybridization.

### Example 13: Measurement of rRNA in bacteria

The present invention can be used to measure bacterial rRNA in a specimen without purifying rRNA. This example illustrates the measurement of a non-mRNA species.

E. coli is cultured in L broth, collected by centrifugation and mixed with 5M GuSCN/.1M EDTA/10 mM dithiothreitol (GED) at a rate of 10⁶ cells/ml. Ten microliters of aliquots of these cells or dilutions in GED are mixed with 2.5 ul of a solution containing 2x10⁵ cpm (2 ng) of a ³²P labeled RNA probe complementary to E. coli rRNA. The mixtures are incubated at 26^{o}C for 5 hours, then are processed by the RNAase/TCA assay described in Example 15. With this procedure a hybridization signal will increase with increasing numbers of E. coli, being in the range of 10 cpm per bacterium, resulting in a hybridization signal of 100,000 cpm with the undiluted sample.

In contrast, essentially no hybridization (200-500 cpm) will occur with probes which are not complementary with E. coli RNA.

It will be obvious to those skilled in the art that, provided a suitable probe is available, the present invention can be used to detect and quantitate any species of RNA, not only the genomic RNA, mRNA, and rRNA depicted in these examples.

## Claims (Claims for the following Contracting State(s): BE, CH, DE, FR, GB, IT, LI, LU, NL, SE)

1. A method of detecting and/or quantifying target nucleic acid in a biological sample, the method comprising:
A. solubilizing the nucleic acid of the biological sample by contacting the biological sample with a chaotropic salt, whereby a solution of solubilized nucleic acid is produced;
B. incubating this solution of solubilized nucleic acid with at least one nucleic acid probe complementary to at least a portion of the solubilized nucleic acid, under conditions which promote molecular hybridization between the probe and the solubilized nucleic acid; and
C. detecting the molecular hybridization.

2. A method as claimed in Claim 1 wherein the solubilized nucleic acid is immobilized.

3. A method as claimed in Claim 1 or 2 wherein the nucleic acid probe is in solution.

4. A method as claimed in any of Claims 1 to 3 wherein the nucleic acid is RNA or DNA and/or the molecular hybridization is DNA-DNA hybridization, DNA-RNA hybridization, or RNA-RNA hybridization.

5. A method as claimed in any of Claims 1 to 4 wherein the chaotropic salt is an alkali metal perchlorate, iodide, trifluoroacetate, trichloroacetate or thiocyanate.

6. A method as claimed in any of Claims 1 to 4 wherein the chaotropic salt is sodium iodide, sodium perchlorate, potassium iodide, sodium thiocyanate, potassium thiocyanate, guanidine thiocyanate, sodium trichloroacetate or sodium trifluoroacetate.

7. A method as claimed in any of Claims 1 to 6 wherein the solubilized DNA is heated to denature the solubilized DNA prior to the incubation step.

8. A method as claimed in any of Claims 1 to 7 wherein the portion of the solubilized nucleic acid is the coding region of the solubilized nucleic acid.

9. A method as claimed in any of Claims 1 to 8 wherein the hybridization is conducted at a temperature in the range of 20° to 40°C.

10. A method as claimed in any of Claims 1 to 9 wherein the nucleic acid probe is complementary to the coding region of the solubilized nucleic acid.

11. A method as claimed in any of Claims 2 to 10 wherein the probe is immobilized on a membrane containing nitrocellulose or nylon.

12. A method as claimed in any of Claims 2 to 11 wherein the probe is immobilized by:
A. contacting the probe with an immobilizing material; and
B. blocking the remaining active sites on the immobilizing material.

13. A method as claimed in any of Claims 1 to 12 wherein the chaotropic salt contains at least one nucleic acid probe.

14. A method as claimed in any of Claims 1 to 13 wherein at least one nucleic acid probe hybridizes to at least a fragment of the HIV viral RNA.

15. A kit suitable for conducting a method according to claim 1, comprising a carrier being compartmentalized to receive at least two container means therein,
one of said container means comprising a chaotropic salt; and
a second container means comprising at least one nucleic acid probe.

16. A kit as claimed in Claim 15 wherein at least one nucleic acid probe is labeled and/or the chaotropic salt is in solution.

17. A kit as claimed in Claim 15 or 16 wherein there is at least one additional container means comprising a positive control biological sample, a negative control biological sample, a standard biological sample, a blocking agent, a nuclease, or a detergent.

18. A kit as claimed in Claim 16 or 17 wherein the chaotropic salt and at least one nucleic acid probe are contained in the same container means.

## Claims (Claims for the following Contracting State(s): AT)

1. A method of detecting and/or quantifying target nucleic acid in a biological sample, the method comprising:
A. solubilizing the nucleic acid of the biological sample by contacting the biological sample with a chaotropic salt, whereby a solution of solubilized nucleic acid is produced;
B. incubating this solution of solubilized nucleic acid with at least one nucleic acid probe complementary to at least a portion of the solubilized nucleic acid, under conditions which promote molecular hybridization between the probe and the solubilized nucleic acid; and
C. detecting the molecular hybridization.

2. A method as claimed in Claim 1 wherein the solubilized nucleic acid is immobilized.

3. A method as claimed in Claim 1 or 2 wherein the nucleic acid probe is in solution.

4. A method as claimed in any of Claims 1 to 3 wherein the nucleic acid is RNA or DNA and/or the molecular hybridization is DNA-DNA hybridization, DNA-RNA hybridization, or RNA-RNA hybridization.

5. A method as claimed in any of Claims 1 to 4 wherein the chaotropic salt is an alkali metal perchlorate, iodide, trifluoroacetate, trichloroacetate or thiocyanate.

6. A method as claimed in any of Claims 1 to 4 wherein the chaotropic salt is sodium iodide, sodium perchlorate, potassium iodide, sodium thiocyanate, potassium thiocyanate, guanidine thiocyanate, sodium trichloroacetate or sodium trifluoroacetate.

7. A method as claimed in any of Claims 1 to 6 wherein the solubilized DNA is heated to denature the solubilized DNA prior to the incubation step.

8. A method as claimed in any of Claims 1 to 7 wherein the portion of the solubilized nucleic acid is the coding region of the solubilized nucleic acid.

9. A method as claimed in any of Claims 1 to 8 wherein the hybridization is conducted at a temperature in the range of 20° to 40°C.

10. A method as claimed in any of Claims 1 to 9 wherein the nucleic acid probe is complementary to the coding region of the solubilized nucleic acid.

11. A method as claimed in any of Claims 2 to 10 wherein the probe is immobilized on a membrane containing nitrocellulose or nylon.

12. A method as claimed in any of Claims 2 to 11 wherein the probe is immobilized by:
A. contacting the probe with an immobilizing material; and
B. blocking the remaining active sites on the immobilizing material.

13. A method as claimed in any of Claims 1 to 12 wherein the chaotropic salt contains at least one nucleic acid probe.

14. A method as claimed in any of Claims 1 to 13 wherein at least one nucleic acid probe hybridizes to at least a fragment of the HIV viral RNA.

## Patentansprüche (Patentansprüche für folgende(n) Vertragsstaat(en): BE, CH, DE, FR, GB, IT, LI, LU, NL, SE)

1. Verfahren zum Nachweisen und/oder mengenmäßigen Bestimmen einer Zielnukleinsäure in einer biologischen Probe, wobei das Verfahren
A. das Löslichmachen der Nukleinsäure der biologischen Probe durch Zusammenbringen der biologischen Probe mit einem chaotropen Salz, wodurch eine Lösung einer in Lösung gebrachten Nukleinsäure hergestellt wird,
B. das Inkubieren dieser Lösung einer in Lösung gebrachten Nukleinsäure mit wenigstens einer Nukleinsäuresonde, die zu wenigstens einem Teil der in Lösung gebrachten Nukleinsäure komplementär ist, unter Bedingungen, welche die molekulare Hybridisierung zwischen der Sonde und der in Lösung gebrachten Nukleinsäure fördern, und
C. das Nachweisen der molekularen Hybridisierung umfaßt.

2. Verfahren wie in Anspruch 1 beansprucht, wobei die in Lösung gebrachte Nukleinsäure immobilisiert ist.

3. Verfahren wie in Anspruch 1 oder 2 beansprucht, wobei sich die Nukleinsäuresonde in Lösung befindet.

4. Verfahren wie in einem der Ansprüche 1 bis 3 beansprucht, wobei die Nukleinsäure RNA oder DNA ist und/oder die molekulare Hybridisierung eine DNA-DNA-Hybridisierung, DNA-RNA-Hybridisierung oder RNA-RNA-Hybridisierung ist.

5. Verfahren wie in einem der Ansprüche 1 bis 4 beansprucht, wobei das chaotrope Salz ein Alkalimetallperchlorat, -iodid, -trifluoracetat, -trichloracetat oder -thiocyanat ist.

6. Verfahren wie in einem der Ansprüche 1 bis 4 beansprucht, wobei das chaotrope Salz Natriumiodid, Natriumperchlorat, Kaliumiodid, Natriumthiocyanat, Kaliumthiocyanat, Guanidinthiocyanat, Natriumtrichloracetat oder Natriumtrifluoracetat ist.

7. Verfahren wie in einem der Ansprüche 1 bis 6 beansprucht, wobei die in Lösung gebrachte DNA erhitzt wird, um die in Lösung gebrachte DNA vor dem Inkubationsschritt zu denaturieren.

8. Verfahren wie in einem der Ansprüche 1 bis 7 beansprucht, wobei der Abschnitt der in Lösung gebrachten Nukleinsäure die Kodierungsregion der in Lösung gebrachten Nukleinsäure ist.

9. Verfahren wie in einem der Ansprüche 1 bis 8 beansprucht, wobei die Hybridisierung bei einer Temperatur im Bereich von 20° bis 40°C ausgeführt wird.

10. Verfahren wie in einem der Ansprüche 1 bis 9 beansprucht, wobei die Nukleinsäuresonde zur Kodierungsregion der in Lösung gebrachten Nukleinsäure komplementär ist.

11. Verfahren wie in einem der Ansprüche 2 bis 10 beansprucht, wobei die Sonde an einer Membrane immobilisiert ist, die Nitrocellulose oder Nylon enthält.

12. Verfahren wie in einem der Ansprüche 2 bis 11 beansprucht, wobei die Sonde durch
A. In-Berührung-bringen der Sonde mit einem immobilisierenden Material und
B. Blockieren der verbleibenden aktiven Stellen auf dem immobilisierenden Material
immobilisiert wird.

13. Verfahren wie in einem der Ansprüche 1 bis 12 beansprucht, wobei das chaotrope Salz wenigstens eine Nukleinsäuresonde enthält.

14. Verfahren wie in einem der Ansprüche 1 bis 13 beansprucht, wobei wenigstens eine Nukleinsäuresonde zu wenigstens einem HIV-Virus-RNA-Fragment hybridisiert.

15. Kit, der zum Ausführen eines Verfahrens gemäß Anspruch 1 geeignet ist, welcher einen abgeteilten Träger zum Aufnehmen wenigstens zweier Behältermittel darin umfaßt, wobei eines der Behältermittel ein chaotropes Salz umfaßt und ein zweites Behältermittel wenigstens eine Nukleinsäuresonde umfaßt.

16. Kit wie in Anspruch 15 beansprucht, in dem wenigstens eine Nukleinsäuresonde markiert ist und/oder sich das chaotrope Salz in Lösung befindet.

17. Kit wie in Anspruch 15 oder 16 beansprucht, in dem sich wenigstens ein zusätzliches Behältermittel befindet, das eine positive biologische Kontrollprobe, eine negative biologische Kontrollprobe, eine biologische Standardprobe, ein Blockierungsmittel, eine Nuklease oder ein Detergens umfaßt.

18. Kit wie in Anspruch 16 oder 17 beansprucht, in dem das chaotrope Salz und wenigstens eine Nukleinsäuresonde in demselben Behältermittel enthalten sind.

## Patentansprüche (Patentansprüche für folgende(n) Vertragsstaat(en): AT)

1. Verfahren zum Nachweisen und/oder mengenmäßigen Bestimmen einer Zielnukleinsäure in einer biologischen Probe, wobei das Verfahren
A. das Löslichmachen der Nukleinsäure der biologischen Probe durch Zusammenbringen der biologischen Probe mit einem chaotropen Salz, wodurch eine Lösung einer in Lösung gebrachten Nukleinsäure hergestellt wird,
B. das Inkubieren dieser Lösung einer in Lösung gebrachten Nukleinsäure mit wenigstens einer Nukleinsäuresonde, die zu wenigstens einem Teil der in Lösung gebrachten Nukleinsäure komplementär ist, unter Bedingungen, welche die molekulare Hybridisierung zwischen der Sonde und der in Lösung gebrachten Nukleinsäure fördern, und
C. das Nachweisen der molekularen Hybridisierung umfaßt.

2. Verfahren wie in Anspruch 1 beansprucht, wobei die in Lösung gebrachte Nukleinsäure immobilisiert ist.

3. Verfahren wie in Anspruch 1 oder 2 beansprucht, wobei sich die Nukleinsäuresonde in Lösung befindet.

4. Verfahren wie in einem der Ansprüche 1 bis 3 beansprucht, wobei die Nukleinsäure RNA oder DNA ist und/oder die molekulare Hybridisierung eine DNA-DNA-Hybridisierung, DNA-RNA-Hybridisierung oder RNA-RNA-Hybridisierung ist.

5. Verfahren wie in einem der Ansprüche 1 bis 4 beansprucht, wobei das chaotrope Salz ein Alkalimetallperchlorat, -iodid, -trifluoracetat, -trichloracetat oder -thiocyanat ist.

6. Verfahren wie in einem der Ansprüche 1 bis 4 beansprucht, wobei das chaotrope Salz Natriumiodid, Natriumperchlorat, Kaliumiodid, Natriumthiocyanat, Kaliumthiocyanat, Guanidinthiocyanat, Natriumtrichloracetat oder Natriumtrifluoracetat ist.

7. Verfahren wie in einem der Ansprüche 1 bis 6 beansprucht, wobei die in Lösung gebrachte DNA erhitzt wird, um die in Lösung gebrachte DNA vor dem Inkubationsschritt zu denaturieren.

8. Verfahren wie in einem der Ansprüche 1 bis 7 beansprucht, wobei der Abschnitt der in Lösung gebrachten Nukleinsäure die Kodierungsregion der in Lösung gebrachten Nukleinsäure ist.

9. Verfahren wie in einem der Ansprüche 1 bis 8 beansprucht, wobei die Hybridisierung bei einer Temperatur im Bereich von 20° bis 40°C ausgeführt wird.

10. Verfahren wie in einem der Ansprüche 1 bis 9 beansprucht, wobei die Nukleinsäuresonde zur Kodierungsregion der in Lösung gebrachten Nukleinsäure komplementär ist.

11. Verfahren wie in einem der Ansprüche 2 bis 10 beansprucht, wobei die Sonde an einer Membrane immobilisiert ist, die Nitrocellulose oder Nylon enthält.

12. Verfahren wie in einem der Ansprüche 2 bis 11 beansprucht, wobei die Sonde durch
A. In-Berührung-bringen der Sonde mit einem immobilisierenden Material und
B. Blockieren der verbleibenden aktiven Stellen auf dem immobilisierenden Material
immobilisiert wird.

13. Verfahren wie in einem der Ansprüche 1 bis 12 beansprucht, wobei das chaotrope Salz wenigstens eine Nukleinsäuresonde enthält.

14. Verfahren wie in einem der Ansprüche 1 bis 13 beansprucht, wobei wenigstens eine Nukleinsäuresonde zu wenigstens einem HIV-Virus-RNA-Fragment hybridisiert.

## Revendications (Revendications pour l'(les) Etat(s) contractant(s) suivant(s): BE, CH, DE, FR, GB, IT, LI, LU, NL, SE)

1. Procédé de détection et/ou de quantification d'un acide nucléique ciblé dans un échantillon biologique, ce procédé consistant à :
A. solubiliser l'acide nucléique de l'échantillon biologique en amenant en contact l'échantillon biologique avec un sel chactropique, de manière à produire une solution d'acide nucléique solubilisé ;
B. incuber cette solution d'acide nucléique solubilisé avec au moins une sonde d'acide nucléique complémentaire d'au moins une partie de l'acide nucléique solubilisé, dans des conditions qui provoquent une hybridation moléculaire entre la sonde et l'acide nucléique solubilisé ; et
C. détecter l'hybridation moléculaire.

2. Procédé selon la revendication 1, caractérisé en ce que l'acide nucléique solubilisé est immobilisé.

3. Procédé selon la revendication 1 ou 2, caractérisé en ce que la sonde d'acide nucléique est en solution.

4. Procédé selon l'une quelconque des revendications 1 à 3, caractérisé en ce que l'acide nucléique est de l'ARN ou de l'ADN et/ou en ce que l'hybridation moléculaire est une hybridation d'ADN-ADN, une hybridation d'ADN-ARN, ou une hybridation d'ARN-ARN.

5. Procédé selon l'une quelconque des revendications 1 à 4, caractérisé en ce que le sel chaotropique est un perchlorate, un iodure, un trifluoroacétate, un trichloroacétate ou un thiocyanate d'un métal alcalin.

6. Procédé selon l'une quelconque des revendications 1 à 4, caractérisé en ce que le sel chaotropique est de l'iodure de sodium, du perchlorate de sodium, de l'iodure de potassium, du thiocyanate de sodium, du thiocyanate de potassium, du thiocyanate de guanidine, du trichloroacétate de sodium ou du trifluoroacétate de sodium.

7. Procédé selon l'une quelconque des revendications 1 à 6, caractérisé en ce que l'ADN solubilisé est chauffé pour dénaturer cet ADN solubilisé avant l'étape d'incubation.

8. Procédé selon l'une quelconque des revendications 1 à 7, caractérisé en ce que la partie de l'acide nucléique solubilisé est la zone de codage de l'acide nucléique solubilisé.

9. Procédé selon l'une quelconque des revendications 1 à 8, caractérisé en ce que l'hybridation est effectuée à une température se situant dans la plage de 20° à 40°C.

10. Procédé selon l'une quelconque des revendications 1 à 9, caractérisé en ce que la sonde d'acide nucléique est complémentaire de la zone de codage de l'acide nucléique solubilisé.

11. Procédé selon l'une quelconque des revendications 2 à 10, caractérisé en ce que la sonde est immobilisée sur une membrane contenant de la nitrocellulose ou du Nylon.

12. Procédé selon l'une quelconque des revendications 2 à 11, caractérisé en ce que la sonde est immobilisée par :
A. mise en contact de la sonde avec un matériau immobilisant ; et
B. blocage des sites actifs restants sur le matériau immobilisant.

13. Procédé selon l'une quelconque des revendications 1 à 12, caractérisé en ce que le sel chaotropique contient au moins une sonde d'acide nucléique.

14. Procédé selon l'une quelconque des revendications 1 à 13, caractérisé en ce qu'au moins une sonde d'acide nucléique s'hybride avec au moins un fragment de l'ADN viral d'HIV.

15. Matériel destiné à mettre en oeuvre un procédé selon la revendication 1, comprenant au moins un porteur compartimenté pour recevoir au moins deux moyens de récipients dans celui-ci,
l'un de ces moyens de récipients comprenant un sel chaotropique ; et
un second moyen de récipient comprenant au moins une sonde d'acide nucléique.

16. Matériel selon la revendication 15, caractérisé en ce qu'au moins une sonde d'acide nucléique est étiquetée et/ou en ce que le sel chaotropique est en solution.

17. Matériel selon l'une des revendications 15 ou 16, caractérisé en ce qu'il existe au moins un moyen de récipient supplémentaire comprenant un échantillon biologique de contrôle positif, un échantillon biologique de contrôle négatif, un échantillon biologique standard, un agent de blocage, une nucléase, ou un détergent.

18. Matériel selon l'une des revendications 16 ou 17, caractérisé en ce que le sel chaotropique et au moins une sonde d'acide nucléique sont contenus dans le même moyen de récipient.

## Revendications (Revendications pour l'(les) Etat(s) contractant(s) suivant(s): AT)

1. Procédé de détection et/ou de quantification d'un acide nucléique ciblé dans un échantillon biologique, ce procédé consistant à :
A. solubiliser l'acide nucléique de l'échantillon biologique en amenant en contact l'échantillon biologique avec un sel chaotropique, de manière à produire une solution d'acide nucléique solubilisé ;
B. incuber cette solution d'acide nucléique solubilisé avec au moins une sonde d'acide nucléique complémentaire d'au moins une partie de l'acide nucléique solubilisé, dans des conditions qui provoquent une hybridation moléculaire entre la sonde et l'acide nucléique solubilisé ; et
C. détecter l'hybridation moléculaire.

2. Procédé selon la revendication 1, caractérisé en ce que l'acide nucléique solubilisé est immobilisé.

3. Procédé selon la revendication 1 ou 2, caractérisé en ce que la sonde d'acide nucléique est en solution.

4. Procédé selon l'une quelconque des revendications 1 à 3, caractérisé en ce que l'acide nucléique est de l'ARN ou de l'ADN et/ou en ce que l'hybridation moléculaire est une hybridation d'ADN-ADN, une hybridation d'ADN-ARN, ou une hybridation d'ARN-ARN.

5. Procédé selon l'une quelconque des revendications 1 à 4, caractérisé en ce que le sel chaotropique est un perchlorate, un iodure, un trifluoroacétate, un trichloroacétate ou un thiocyanate d'un métal alcalin.

6. Procédé selon l'une quelconque des revendications 1 à 4, caractérisé en ce que le sel chaotropique est de l'iodure de sodium, du perchlorate de sodium, de l'iodure de potassium, du thiocyanate de sodium, du thiocyanate de potassium, du thiocyanate de guanidine, du trichloroacétate de sodium ou du trifluoroacétate de sodium.

7. Procédé selon l'une quelconque des revendications 1 à 6, caractérisé en ce que l'ADN solubilisé est chauffé pour dénaturer cet ADN solubilisé avant l'étape d'incubation.

8. Procédé selon l'une quelconque des revendications 1 à 7, caractérisé en ce que la partie de l'acide nucléique solubilisé est la zone de codage de l'acide nucléique solubilisé.

9. Procédé selon l'une quelconque des revendications 1 à 8, caractérisé en ce que l'hybridation est effectuée à une température se situant dans la plage de 20° à 40°C.

10. Procédé selon l'une quelconque des revendications 1 à 9, caractérisé en ce que la sonde d'acide nucléique est complémentaire de la zone de codage de l'acide nucléique solubilisé.

11. Procédé selon l'une quelconque des revendications 2 à 10, caractérisé en ce que la sonde est immobilisée sur une membrane contenant de la nitrocellulose ou du Nylon.

12. Procédé selon l'une quelconque des revendications 2 à 11, caractérisé en ce que la sonde est immobilisée par :
A. mise en contact de la sonde avec un matériau immobilisant ; et
B. blocage des sites actifs restants sur le matériau immobilisant.

13. Procédé selon l'une quelconque des revendications 1 à 12, caractérisé en ce que le sel chaotropique contient au moins une sonde d'acide nucléique.

14. Procédé selon l'une quelconque des revendications 1 à 13, caractérisé en ce qu'au moins une sonde d'acide nucléique s'hybride avec au moins un fragment de l'ADN viral d'HIV.
